# EUROPEAN PATENT SPECIFICATION

(11) **EP 1 624 956 B1**
(45) Date of publication and mention of the grant of the patent: **26.06.2013**
(21) Application number: 04751914.5
(22) Date of filing: 12.05.2004
(51) Int. Cl.: B01F 15/02, B01F 11/00, B01F 13/06, A61B 17/88, B01F 13/00

(54) **BONE CEMENT MIXING CARTRIDGE WITH A RELEASABLY LOCKED DRIVE PISTON, THE DRIVE PISTON CONFIGURED TO BE UNLOCKED BY A DELIVERY DEVICE.**
KARTUSCHE ZUM MISCHEN VON KNOCHENZEMENT MIT EINEM VERRIEGELTEN KOLBEN, DER KOLBEN SO GESTALTET, DASS ER VON EINEM AUSTRAGSGERÄT UNVERRIEGELT WIRD.
CARTOUCHE DE MELANGEAGE DE CIMENT POUR OS, AVEC UN PISTON A VERROULLAGE AMOVIBLE, LE PISTON ETANT CONFIGURE POUR ETRE DEVERROUILLE PAR LE DISPOSITIF D'ADMINISTRATION.

(30) Priority: 12.05.2003 US 469651 P; 18.11.2003 US 520877 P
(43) Date of publication of application: 15.02.2006
(73) Proprietor: Stryker Corporation, Kalamazoo, MI 49002 (US)
(72) Inventor: HENNIGES, Bruce, D., Galesburg, MI 49053 (US); TAGUE, Christopher, M., Delton, MI 49046 (US); COFFEEN, Jared, P., Paw Paw, MI 49079 (US); BROCKMAN, Christopher S., Kalamazoo, MI 49008 (US); PROULX, Marshall, K., Portage, MI 49002 (US)
(74) Representative: Röthinger, Rainer
(86) International application number: PCT/US2004/014749
(87) International publication number: WO 2004/100771

(56) References cited:
- EP-A2- 1 005 900
- US-A- 4 277 184
- US-A- 4 371 094
- US-A- 5 071 040
- US-A- 5 876 116
- US-A1- 2003 067 837
- US-B1- 6 260 737
- US-B1- 6 406 175

## Description

### RELATED APPLICATIONS

### FIELD OF THE INVENTION

The present invention generally relates to a bone cement mixing and delivery system. More specifically, the present invention relates to a mixing cartridge for receiving liquid and powder components of bone cement to be mixed, a mixing device for mixing the components, and a delivery gun for discharging the bone cement from the mixing cartridge into an anatomical site of a patient.

### BACKGROUND OF THE INVENTION

Bone cement mixing and delivery systems are well known for mixing liquid and powder components of bone cement and delivering the prepared bone cement to an anatomical site during various surgical procedures. Bone cement is particularly useful in orthopedic procedures in which a prosthetic device is fixed to a bone or joint structure to improve the strength, rigidity, and movement of the structure. In a total hip arthroplasty (THA) procedure, in which a hip joint is replaced with a prosthetic device, bone cement is used to fix the prosthetic device in place in a medullary canal of a femur.

Typically, the bone cement is prepared in a mixing cartridge. The mixing cartridge includes a cylinder having proximal and distal ends with a mixing chamber defined between the ends. The mixing cartridge further includes a cap covering the proximal end of the cylinder and a piston disposed in the distal end of the cylinder such that the mixing chamber is further defined between the cap and the piston. The piston may be releasably secured in a locked position in the cylinder by a cotter pin. The cap supports a mixing device, i.e., a mixing shaft and blade, for mixing the liquid and powder components of the bone cement in the mixing chamber.

Once the bone cement is mixed, the mixing cartridge is prepared for inserting into a delivery gun to discharge the bone cement. This may include disengaging the mixing shaft and coupling a nozzle to the cap to provide a discharge point for the bone cement. At the same time, the piston is released from the locked position in the distal end of the cylinder by pulling the cotter pin. This allows the piston to be driven by the delivery gun through the mixing chamber to discharge the bone cement from the nozzle. An alternative solution for securing and releasing the piston is shown in United States Patent No. 5,328,262 to Lidgren et aL

In Lidgren et al., the piston is releasably secured in the locked position in the distal end of the cylinder by a gripping portion in the form of a flange, which extends along only a portion of an inner periphery of the cylinder. The piston in Lidgren et al. has a corresponding gripping portion in the form of an outwardly directed lip that protrudes behind the flange. The lip defines a groove with an outer surface of the piston to receive the flange. To release the piston from the locked position, the flange is rotated through the groove until the flange has been rotated past the lip. Lidgren et al. discloses a base that is used to secure the piston from rotation while a user rotates the cylinder relative to the piston to release the piston from the locked position. This method of releasing the piston from the locked position, much like pulling the cotter pin, requires additional manipulation by a user.

Once the piston is released from the locked position, the mixing cartridge is inserted into the delivery gun. A typical delivery gun includes a ram disk that engages the piston and drives the piston through the mixing chamber to discharge the bone cement from the nozzle. The delivery gun includes a cradle for supporting the mixing cartridge and a casing for supporting a drive rod that engages the ram disk and advances the ram disk to drive the piston. The drive rod includes a plurality of teeth and a pawl member engages the teeth to advance the drive rod. A trigger supports the pawl member and the casing rotatably supports the trigger. Actuation of the trigger relative to the casing urges the pawl member against the teeth to advance the drive rod.

An example of such a delivery gun is illustrated in United States Patent No. 5,431,654 to Nic. In the '654 patent to Nic, two pawl members are used to independently advance the drive rod and the ram disk. The pawl members provide high speed/low force and low speed/high force advancement of the drive rod. A switch is used to select between the speeds. When high speed is selected, both pawl members engage the drive rod, while only the high-speed pawl member actually advances the drive rod. When low speed is selected, the high-speed pawl member is isolated from the teeth such that only the low speed pawl member engages the teeth to advance the drive rod. However, in Nic, the trigger directly supports each of the pawl members which results in a low mechanical advantage to advance the drive rod and ram disk.

EP 1 005 900 A2 discloses a mixing cartridge according to the preamble of present claim 1.

US 6,406,175 B1 discloses a device for preparing and injecting a polymeric bone cement under sterile, isobaric and isovolumic conditions. The device includes a cylindrical vessel holding a volume of polymeric powder and a disk agitator mounted on a shaft passing through an aperture in a proximal end wall of the vessel and extending beyond the agitator through a delivery port in an opposite wall of the vessel. A piston can be selectively coupled to the disk in order to inject the contents of the vessel through the outlet port. The cement is ejected by braking off a distal, tubular section of the shaft and mounting the broken off section over an external bushing surrounding the outlet port and moving the piston toward the outlet port to expel the contents of the vessel.

### BRIEF SUMMARY OF THE INVENTION

According to the present invention, a mixing cartridge according to claim 1 adapted for use with a delivery device for discharging bone cement from the mixing cartridge is provided. The dependent claims relate to advantageous configurations of the mixing cartridge and the use of the mixing cartridge in a bone cement system.

A mixing cartridge for receiving liquid and powder components of bone cement to be mixed for medical use is disclosed that comprises a cylinder having proximal and distal ends with a mixing chamber defined therebetween. The cylinder includes a cylinder wall extending between the ends about a longitudinal axis of the cylinder. A piston is disposed in the cylinder at the distal end such that the mixing chamber is further defined between the proximal end and the piston. A locking member is coupled to the piston to lock the piston in the distal end. The locking member includes a male portion engaging a female portion in the cylinder wall to place the piston in a locked position at the distal end of the cylinder. The locking member includes a resilient portion for biasing the male portion into mating engagement with the female portion. The piston remains in the locked position at the distal end of the cylinder while mixing the liquid and powder components.

One advantage of the mixing cartridge is the conveniently positioned locking member used to lock the piston in the distal end. By using the resilient portion to bias the male portion into mating engagement with the female portion, a user can easily release the piston from the locked position by mechanically acting against the bias of the resilient portion to disengage the male and female portions. user can easily release the piston from the locked position by mechanically acting against the bias of the resilient portion to disengage the male and female portions.

A delivery gun is also disclosed for discharging the bone cement from the cartridge once the bone cement is prepared. The delivery gun comprises a casing for supporting the cartridge. A drive mechanism is supported by the casing and advanceable relative to the casing to force the bone cement from the cartridge. The casing pivotally supports a trigger operatively connected to the drive mechanism to advance the drive mechanism upon actuation of the trigger to force the bone cement from the cartridge. A linkage system works in conjunction with the trigger to advance the drive mechanism. The linkage system comprises a first link pivotally connected to the casing and a second link interconnecting the first link and the trigger such that actuating the trigger moves the second link and the first link to advance the drive mechanism.

An advantage of the delivery gun is the use of the linkage system to increase the mechanical advantage needed to successfully advance the drive mechanism and force the bone cement from the cartridge while minimizing fatigue to a user of the delivery gun.

In one aspect of the delivery gun, the drive mechanism includes a drive rod and gripper plates to advance the drive rod. The gripper plates frictionally engage the drive rod to advance the drive rod when the trigger is actuated. In one embodiment, the gripper plates include mating pegs and notches to align adjacent gripper plates. In another embodiment, the gripper plates are coated to increase lubricity and corrosion resistance thereof.

In another aspect of the delivery gun, the drive mechanism includes a drive rod and first and second pawl members to advance the drive rod. In one embodiment, the second pawl member is movable into engagement with teeth on the drive rod for high-speed advancement of the drive rod and out from engagement with the teeth for low-speed advancement. During low-speed advancement, only the first pawl member engages the teeth to advance the drive rod. During high-speed advancement, both pawl members engage the teeth, but only the second pawl member works to advance the drive rod.

A bone cement mixing and delivery system is also disclosed. The mixing and delivery system includes the cartridge and the delivery gun. In this aspect, the locking member includes a release button to release the piston from the locked position. At the same time, the delivery gun includes a release mechanism integrated into the drive mechanism to engage the release button. When the cartridge is placed into the cradle of the delivery gun, the drive mechanism is advanced and the release mechanism engages the release button to release the piston from the locked position. This configuration reduces the number of steps typically associated with releasing the piston. By incorporating the release mechanism into the drive mechanism, when the drive mechanism is advanced, the piston is automatically released.

A bone cement loading system for receiving the liquid and powder components of the bone cement is also disclosed. The loading system includes the cylinder with the piston locked in the distal end. A base defining a cavity is provided for receiving and securing the distal end of the cylinder. A funnel is provided for coupling to the proximal end of the cylinder to channel the powder component of the bone cement into the mixing chamber. The funnel has a proximal end with an oblong oval-shaped periphery to facilitate loading of the powder component of the bone cement into the mixing chamber and a distal end with a circular periphery for snugly fitting into the proximal end of the cylinder. One particular advantage to this loading system is the use of the oblong oval-shaped funnel. The shape of the funnel reduces any mess typically associated with filling the mixing chamber with powder.

A bone cement mixing system comprising the mixing cartridge and a mixing shaft and blade is also disclosed. The blade is coupled to the mixing shaft and disposed in the mixing chamber for rotating with the mixing shaft about the longitudinal axis to mix the liquid and powder components of the bone cement. The blade includes a center hub coupled to the mixing shaft and an outer ring extending from the center hub. The outer ring forms an acute angle with the longitudinal axis of between twenty and seventy degrees to ensure adequate mixing of the bone cement in the mixing chamber.

A method of mixing the liquid and powder components of the bone cement in the mixing chamber is also disclosed. The method includes using a rotary power tool connected to a portion of the mixing shaft extending outside of the mixing chamber to mix the liquid and powder components of the bone cement. The blade is disposed in the mixing chamber while being operatively connected to the portion of the mixing shaft extending outside of the mixing chamber. In the method, the rotary power tool is first connected to the portion of the mixing shaft extending outside of the mixing chamber. Then the rotary power tool is actuated to rotate the blade and mix the liquid and powder components of the bone cement. At the same time, the rotary power tool is axially displaced relative to the mixing cartridge to completely mix the liquid and powder components of the bone cement. Once mixing is complete, the operative connection between the blade and the portion of the mixing shaft extending outside of the mixing chamber is removed.

### BRIEF DESCRIPTION OF THE SEVERAL VIEWS OF THE DRAWINGS

Advantages of the present invention will be readily appreciated as the same becomes better understood by reference to the following detailed description when considered in connection with the accompanying drawings wherein:

Figure 1 is an exploded perspective view of a mixing cartridge of the present invention in combination with a mixing shaft and blade;

Figure 2 is an assembled perspective view of the mixing cartridge with the mixing shaft and blade supported therein;

Figure 3 is an exploded perspective view of a cap of the mixing cartridge;

Figure 4 is a cross-sectional view of the cap of Figure 3 and a partial cross-sectional view of a cylinder of the mixing cartridge to illustrate fitting of the cap to the cylinder;

Figure 5 is an exploded perspective view of the cap and the mixing shaft and blade;

Figure 6 is an assembled perspective view of the cap with the mixing shaft and blade supported therein;

Figure 7 is a perspective view of the blade;

Figure 7A is a side elevational view of the blade of Fig. 7;

Figures 8-8A and 9 are perspective views of alternative blades;

Figure 10 is a an exploded perspective view of the mixing shaft and a latch rod;

Figure 11 is an elevational end view of the mixing shaft and latch rod of Fig. 10;

Figure 12 is a cross-sectional view of the mixing shaft and latch rod of Figs. 10 and 11;

Figure 13 is an exploded perspective view of a release latch coupling the mixing shaft and latch rod;

Figures 14A-14C illustrate the release of the blade from the mixing shaft;

Figure 15 is an exploded perspective view of a piston of the mixing cartridge;

Figure 16 is a cross-sectional view of the piston of Fig. 15;

Figure 17 is a perspective view of an alternative piston of the mixing cartridge;

Figure 18 is a top view of the alternative piston of Fig. 17;

Figure 19 is an exploded perspective view of the cap and a nozzle;

Figure 20 is an assembled perspective view of the cap and nozzle;

Figure 21 is a blown-up view of a locking mechanism of the cap and nozzle;

Figures 22-23 are perspective views of the nozzle;

Figure 24 is a perspective view of a delivery gun illustrating a linkage system of the delivery gun;

Figures 24A-24B illustrate alternative linkage systems;

Figure 25 is an elevational view illustrating release of a locking member securing the piston;

Figure 26 is a partial perspective view of an alternative linkage system and drive mechanism of the delivery gun;

Figure 27 is a partial perspective view of the alternative linkage system and drive mechanism of Fig. 26 employing a striker to prevent freeze-up of the drive mechanism;

Figures 28 is an elevational view of a second alternative linkage system and drive mechanism of the delivery gun in a low-speed position;

Figure 29 is a perspective view of the second alternative linkage system and drive mechanism in the low-speed position;

Figure 30 is an elevational view of the second alternative linkage system and drive mechanism in a high-speed position;

Figure 31 is a perspective view of the second alternative linkage system and drive mechanism in the high-speed position;

Figure 32 is an exploded view of a cylinder of the mixing cartridge and a base and funnel used to fill the cylinder with components of bone cement; and

Figures 33-42 illustrate various steps associated with the present disclosure.

### DETAILED DESCRIPTION OF THE INVENTION

Referring to the Figures, wherein like numerals indicate like or corresponding parts throughout the several views, a bone cement mixing and delivery system is generally shown. The bone cement mixing and delivery system comprises a mixing cartridge **100** for receiving liquid monomer and powdered copolymer components of bone cement to be mixed, a mixing device (mixing shaft **150** and blade **152**) for mixing the components, and a delivery device, e.g., a delivery gun **500,** for discharging the bone cement from the mixing cartridge **100** into an anatomical site (not shown). An exemplary use for the bone cement is to secure a prosthetic device used to replace a joint structure such as in a total hip arthroplasty (THA) procedure.

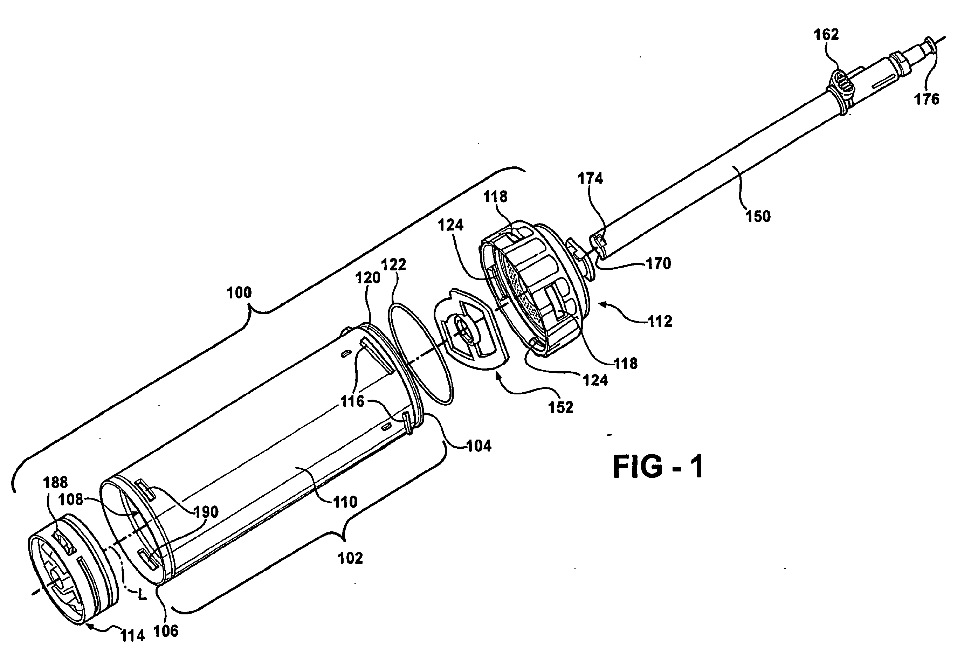

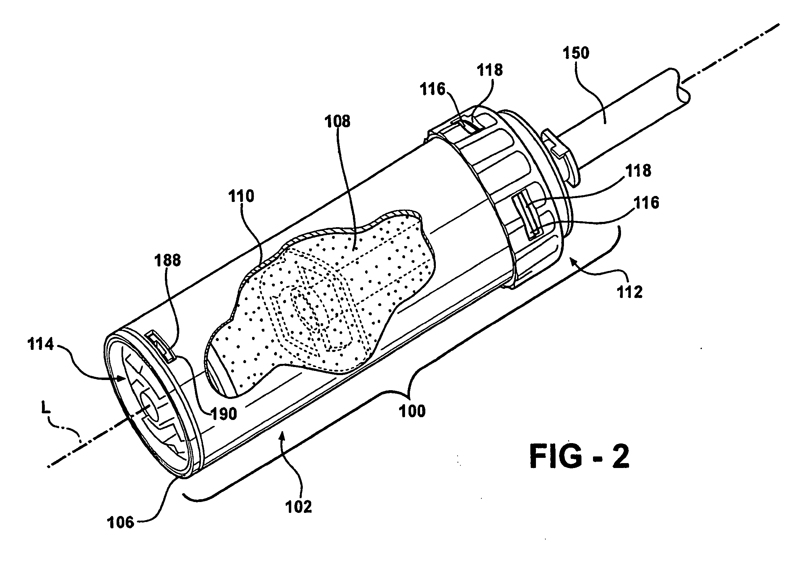

Referring to FIGS. 1 and 2, the bone cement mixing system comprises the mixing cartridge **100** in combination with the mixing shaft **150** and blade **152** used to mix the components of the bone cement in the mixing cartridge **100.** The mixing cartridge **100** includes a cylinder **102** having proximal **104** and distal **106** ends. A mixing chamber **108** is defined between the ends **104, 106.** The cylinder **102** includes a cylinder wall **110** extending between the ends **104, 106,** about a longitudinal axis **L.** A cap **112** is coupled to the cylinder **102** at the proximal end **104** and a piston **114** is disposed in the cylinder **102** at the distal end **106** such that the mixing chamber **108** is further defined between the cap **112** and the piston **114.** The components of the bone cement are placed in the mixing chamber **108** and mixed by the mixing shaft **150** and blade **152,** as will be described further below.

In the preferred embodiment, the cylinder **102** has locking strips **116** disposed on the cylinder wall **110** at the proximal end **104** to insert into locking slots **118** on the cap **112.** Each of the locking strips **116** include a straight portion lying perpendicular relative to the longitudinal axis **L** and an angled portion lying at an angle relative to the straight portion. As should be appreciated, the locking strips **116** and locking slots **118** could be reversed, i.e., the locking strips **116** positioned on the cap **112** and the locking slots **118** defined in the cylinder wall **110.** The locking strips **116** and locking slots **118** are configured to provide quick locking of the cap **112** onto the cylinder **102** with a one-quarter turn of the cap **112.** Those of ordinary skill in the art will appreciate that numerous methods are available for connecting the cap **112** to the cylinder **102,** such as mating threads, snap-fit connections, etc. A groove **120** is formed in the cylinder **102** at the proximal end **104** to seat an o-ring seal **122.** The o-ring seal **122** assists in sealing the cap **112** to the cylinder **102.**

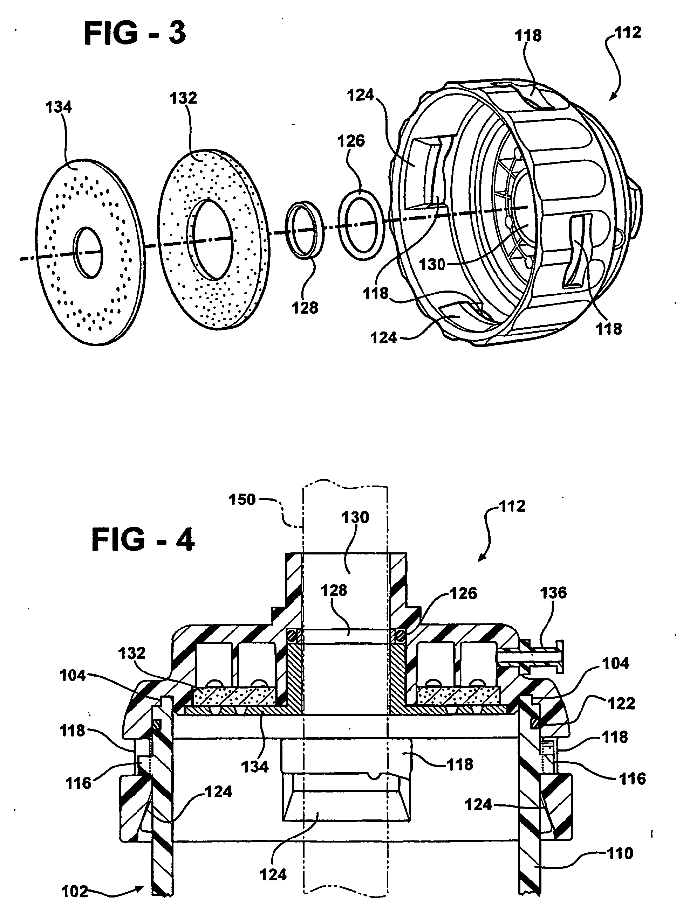

Referring to FIGS. 3-4, the cap **112** includes radially inwardly protruding ramps **124** that lead into the locking slots **118** to facilitate the fit with the locking strips **116** on the cylinder wall **110.** When first placing the cap **112** on the cylinder **102,** the locking strips **116** are positioned between the ramps **124.** As the cap **112** is rotated, the ramps **124** cam the locking strips **116** proximally to urge the proximal end **104** of the cylinder **102** into a sealed relationship with the cap **112,** as shown in FIG. 4 (only a portion of the cylinder wall **110** with two locking strips **116** is shown in FIG. 4 for illustrative purposes). In the preferred embodiment, there are four locking strips **116** and four locking slots **118** to facilitate the sealed relationship between the cap **112** and the cylinder **102.**

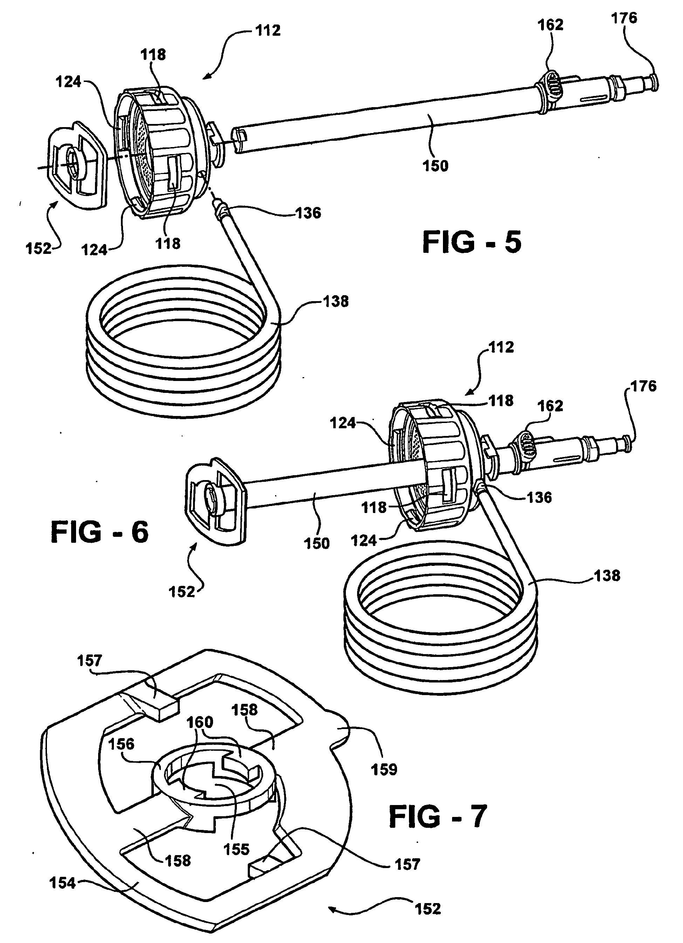

Referring specifically to FIG. 4, an o-ring seal **126** and dynamic seal **128** operate together within an orifice **130** in the cap **112** to movably support and seal to the mixing shaft **150.** The mixing shaft **150** slides through the orifice **130** and the dynamic seal **128** and is movably supported therein. The dynamic seal **128** allows nearly frictionless rotational, as well as axial movement of the mixing shaft **150** within the mixing chamber **108** to mix the liquid and powder components of the bone cement, while maintaining a snug fit within the orifice **130.** A filter **132** and liner **134** are positioned on an interior of the cap **112** to allow a vacuum to be drawn in the mixing chamber **108** by way of a vacuum port **136.** The vacuum port **136** is isolated from the mixing chamber **108** by the filter **132** and liner **134** to prevent fouling of a vacuum pump (not shown). Referring to FIGS. 5-6, a vacuum tube **138** is shown attached to the vacuum port **136** to draw the vacuum in the mixing chamber **108** during mixing.

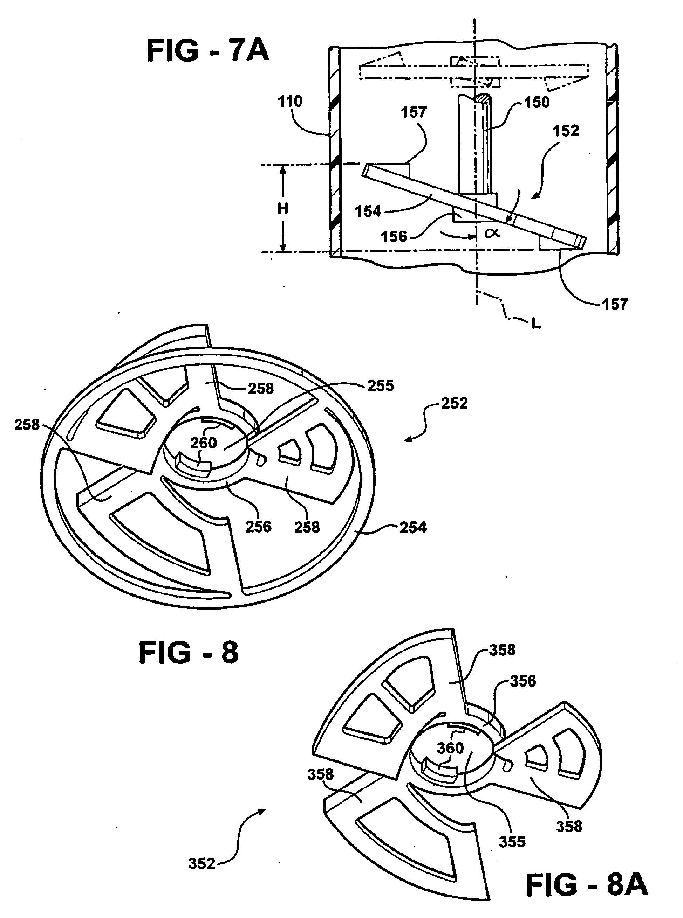

Referring to FIG. 7, the preferred blade **152** used to mix the bone cement is shown. The blade **152** is integrally formed from plastic in one piece and has an outer ring **154** connected to a center hub **156** by vanes **158.** Ears **160** protrude radially inwardly from the center hub **156** to facilitate a releasable connection to the mixing shaft **150.** The releasable connection is described further below. Referring to FIG. 7A, the outer ring **154** forms an acute angle α with the longitudinal axis **L** of the cylinder **102** (which is also a rotational mixing axis of the blade **152).** The acute angle α is important for efficient mixing of the bone cement The acute angle α is preferably between twenty and seventy degrees, and more preferably sixty degrees. The blade **152** has an effective height **H** that is greater than one quarter inch to ensure adequate mixing. Preferably, the effective height **H** of the blade **152** is approximately one half inch.

Referring back to FIG. 7, two radially inwardly protruding fingers **157** are attached to the outer ring **154.** One of the fingers **157** protrudes radially inwardly in a first plane and the other finger **157** protrudes radially inwardly in a second plane spaced from and parallel to the first plane. The center hub **156** is positioned between the planes. The fingers **157** are used to scrape proximal and distal regions of the mixing chamber **108** to ensure complete mixing. A protruding node **159** is also attached to the outer ring **154.** The node **159** protrudes radially outwardly to control spacing between the blade **152** and an inner periphery of the cylinder wall **110** by scraping along the inner periphery of the cylinder wall **110** in the mixing chamber **108.**

FIGS. 8 and 8A illustrate alternative blades **252, 352** that could also be used to mix the bone cement. Each of the blades **152, 252, 352** is designed to flatten at the proximal end **104** of the cylinder **102** adjacent to the cap **112** after the blade **152, 252, 352** is released from the mixing shaft **150** in the mixing chamber **108.** This ensures that the maximum possible amount of bone cement can be discharged from the mixing cartridge **100.** In the case of the preferred blade **152,** the blade **152** is flexible and the outer wall **154** flattens into a plane perpendicular to the longitudinal axis **L** and occupied by the center hub **156,** as illustrated by hidden lines in FIG. 7A. Thus, the effective height **H** is reduced and the acute angle **α** becomes close to ninety degrees. This is accomplished by twisting at the vanes **158.** Spaces **155, 255, 355** formed in the center hub **156, 256, 356** ensure that once the blade **152, 252, 352** is flattened, the bone cement can pass through the blade **152, 252, 352** when discharged from the mixing cartridge **100.** To further facilitate the discharge of the bone cement past the blades **152, 252, 352,** each of the center hubs **156, 256, 356** are sized to partially fit within the aperture **130** defined in the cap **112.**

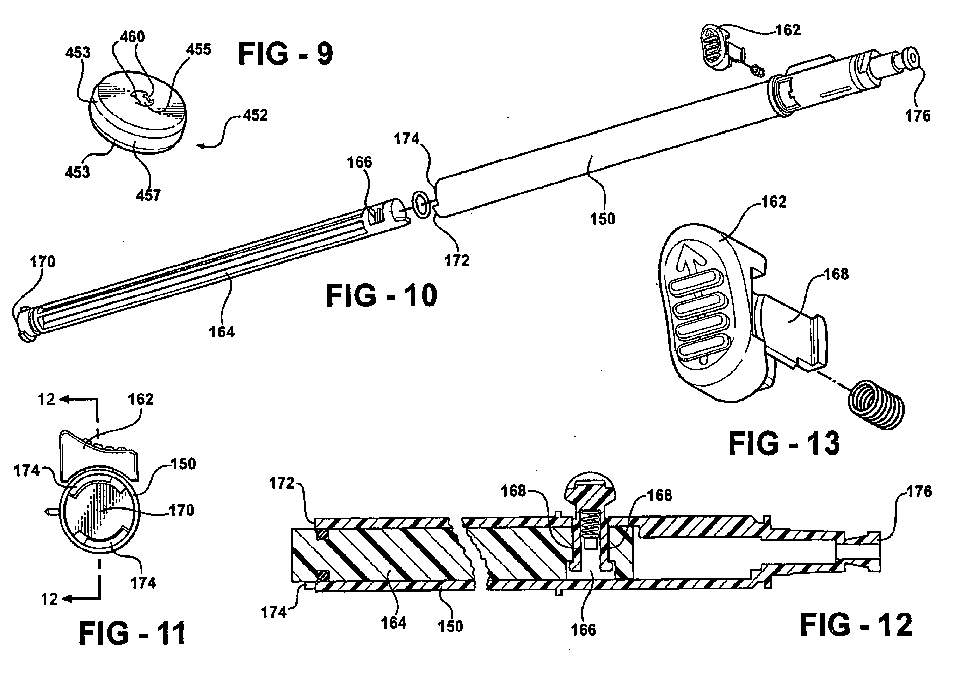

Another alternative blade **452** is shown in FIG. 9. This blade **452** is a relatively thick disk **452** with chamfered ends **453** forming an acute chamfer angle with a sidewall **457.** The chamfer angle is preferably sixty degrees. In the preferred embodiment, the disk is about one half inch thick and about one eighth inch less in diameter than the inner periphery of the cylinder wall **110.** In one embodiment, the inner periphery of the cylinder wall **110** is about two and one quarter inches in diameter. As should be appreciated, the slight distance between the side wall **457** of the disk **452** and the inner periphery of the cylinder wall **110** creates a shear force on the bone cement as the disk **452** is rotated and moved axially in the mixing chamber **108.** The shear force is the force applied to the bone cement to mix the bone cement. This blade **452** also includes a space **455** formed in a center of the disk **452** and ears **460** for releasably attaching to the mixing shaft **150.**

Referring to FIGS. 10-13 the mixing shaft **150** has a release latch **162** for releasing the blade **152** from the mixing shaft **150** once mixing of the bone cement is complete. The release latch **162** moves between a holding position and a releasing position. In the holding position, the blade **152** is secured to the mixing shaft **150** to mix the bone cement in the mixing chamber **108.** In the releasing position, the blade **152** is released from the mixing shaft **150** to remain in the mixing chamber **108** while the mixing shaft **150** is removed from the cap **112** to make way for a nozzle **204,** as will be described further below. The release latch **162** is operatively connected to a latch rod **164,** which latches the blade **152** to the mixing shaft **150** in the holding position. The latch rod **164** defines a split cavity **166** for receiving split legs **168** of the release latch **162** in a snap-fit manner. The latch rod **164** is rotatably supported within the mixing shaft **150.**

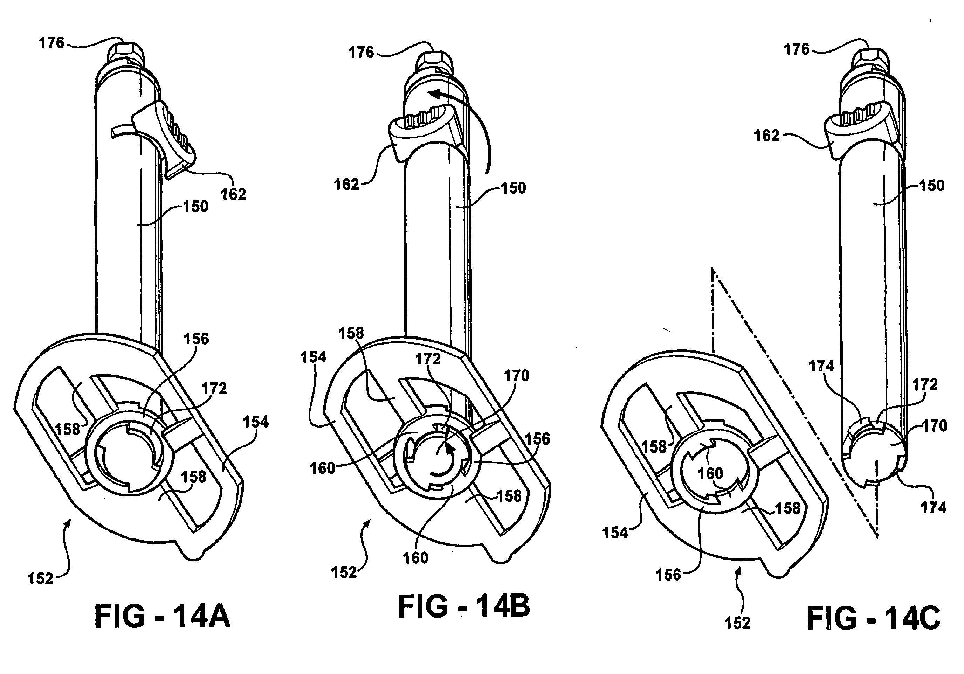

Referring to FIGS. 14A-14C, the transition of the release latch **162** between the holding position and the releasing position is illustrated. Referring first to FIG. 14C, the exposed end **170** of the latch rod **164** is generally "T" shaped The corresponding end **172** of the mixing shaft **150** has opposed notches **174** that are adapted to receive the ears **160** on the center hub **156** of the blade **152.** Initially, the ears **160** are positioned in the notches **174** and the exposed end **170** is positioned over the ears **160** to hold the blade **152** to the mixing shaft **150.** See FIG. 14A. To release the blade **152,** the release latch **162** is depressed and rotated. Rotating the release latch **162** rotates the latch rod **164** with respect to the mixing shaft **150** thus rotating the exposed end **170** away from the ears **160** to release the blade **152.** See FIG. 14B. With the blade **152** released, the mixing shaft **150** is withdrawn from the cap **112** while the blade **152** remains in the mixing chamber **108.**

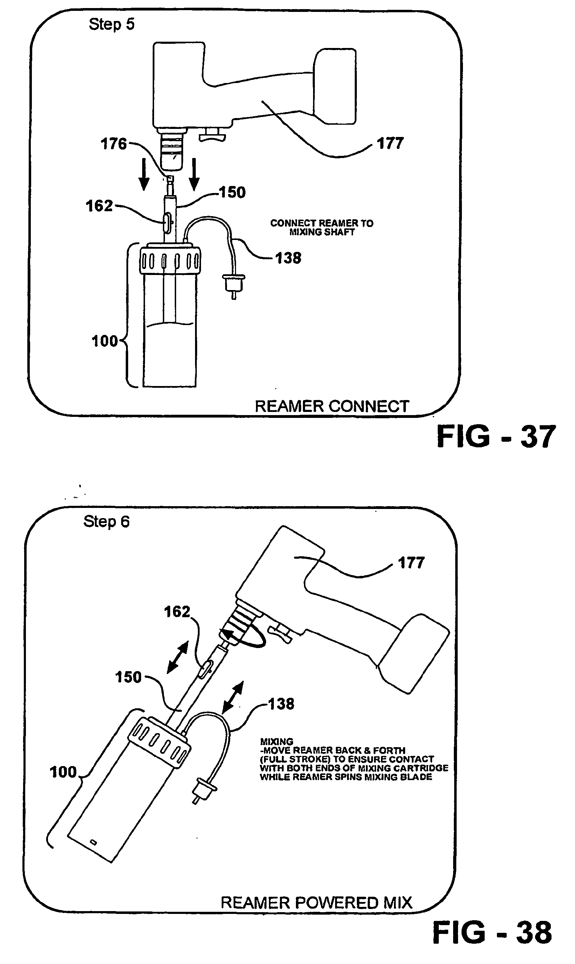

A proximal end **176** of the mixing shaft **150,** which represents a portion of the mixing shaft **150** extending outside of the mixing chamber **108** during mixing, is adapted to engage a rotary power tool **177** (see FIG. 37), such as a reamer drill, used to rotate the mixing shaft **150** and blade **152** and mix the bone cement. The proximal end **176** of the mixing shaft **150** is operatively connected to the blade **152** to transfer the rotation of the rotary power tool **177** to the blade **152.** When the blade **152** is released from the mixing shaft **150,** the operative connection is removed. The operative connection is also removed if the portion of the mixing shaft **150** extending outside of the mixing chamber **108** is severed from the rest of the mixing shaft **150** in the mixing chamber **108,** as in alternative embodiments. A manually operated mixing handle (not shown) could engage the mixing shaft **150** at the proximal end **176** to mix the bone cement in other embodiments.

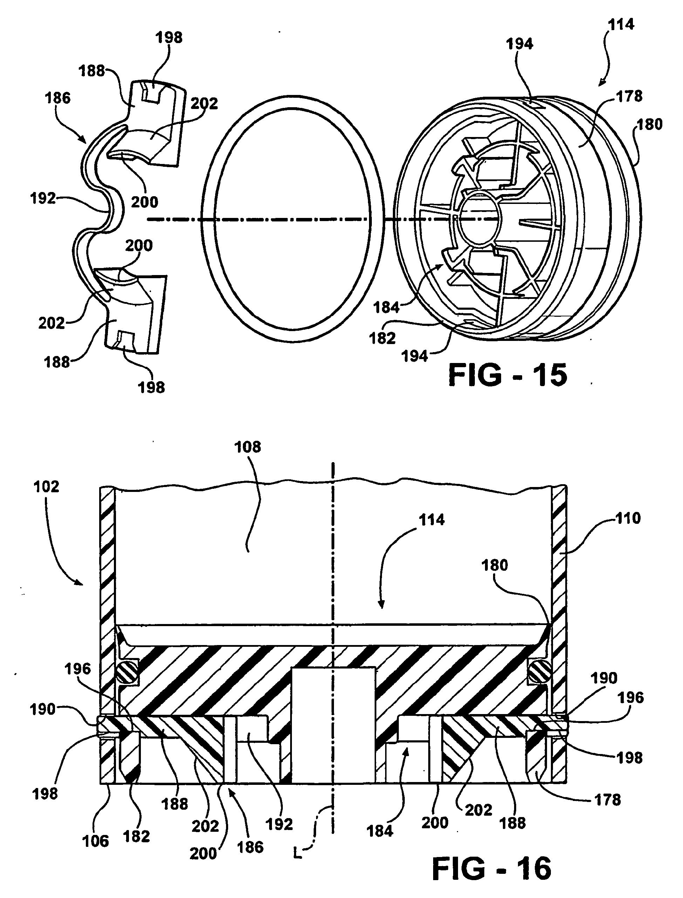

Referring to FIGS. 15-16, the piston **114** is positioned within the distal end **106** of the cylinder **102** to further seal the mixing chamber **108.** The piston **114** has a skirt **178** extending about the inner periphery of the cylinder wall **110.** The piston **114** also includes a proximal end **180** and a distal end **182** defining a cavity **184.**

Referring specifically to FIG. 16, the piston **114** is releasably secured in a locked position in the distal end **106** of the cylinder **102** by a locking member **186.** The locking member **186** is disposed in the cavity **184** and includes diametrically opposed locking tabs **188** protruding into diametrically opposed slots **190** defined in the cylinder wall **110** to secure the piston **114** to the cylinder **102.** It should be appreciated that the slots **190** could be in the form of any suitable female portion, e.g., slot, groove, channel, etc., used for interlocking with a corresponding male portion such as the locking tabs **188.** Furthermore, while the embodiment of FIG. 16 illustrates two-way locking, i.e., the piston **114** being locked from moving proximally and distally, the locking member **186** could also be used for one-way locking, i.e., for preventing only proximal movement of the piston **114.**

The locking member **186** is integrally formed from plastic and a resilient portion **192** of the locking member **186** biases the locking tabs **188** radially outwardly from the longitudinal axis **L** into the slots **190.** The resilient portion **192** is in the form of a thin resilient ribbon **192** acting like a spring and extending is a winding shape between the locking tabs **188.** The locking tabs **188** couple the locking member **186** to the piston **114** by protruding through carrier slots **194** formed in the skirt **178.** In the preferred embodiment, a step **196** protrudes into each of the carrier slots **194** to define a guide for sliding engagement within a channel **198** partially defined in each of the locking tabs **188.** In the locked position, the carrier slots **194** are axially and radially aligned with the slots **190** formed in the cylinder wall **110.**

The piston **114** is locked at the distal end **106** of the cylinder **102** while the liquid and powder components are added and mixed in the mixing cartridge **100.** The piston **114** is released from the locked position after mixing of the bone cement is complete. Release buttons **200,** integrally formed with the locking tabs **188,** are used to release the piston **114** from the locked position. The release buttons **200** are disposed on the locking tabs **188** and protrude distally therefrom. Each of the release buttons **200** includes a cam surface **202** forming an acute angle with the longitudinal axis **L.** The piston **114** is released from the locked position by squeezing the release buttons **200** radially inwardly against the bias of the resilient portion **192** to withdraw the locking tabs **188** from the slots **190.** This action can be performed either manually or mechanically, as will be described further below. After release from the slots **190,** the locking tabs **188** remain coupled to the piston **114** in the carrier slots **194.**

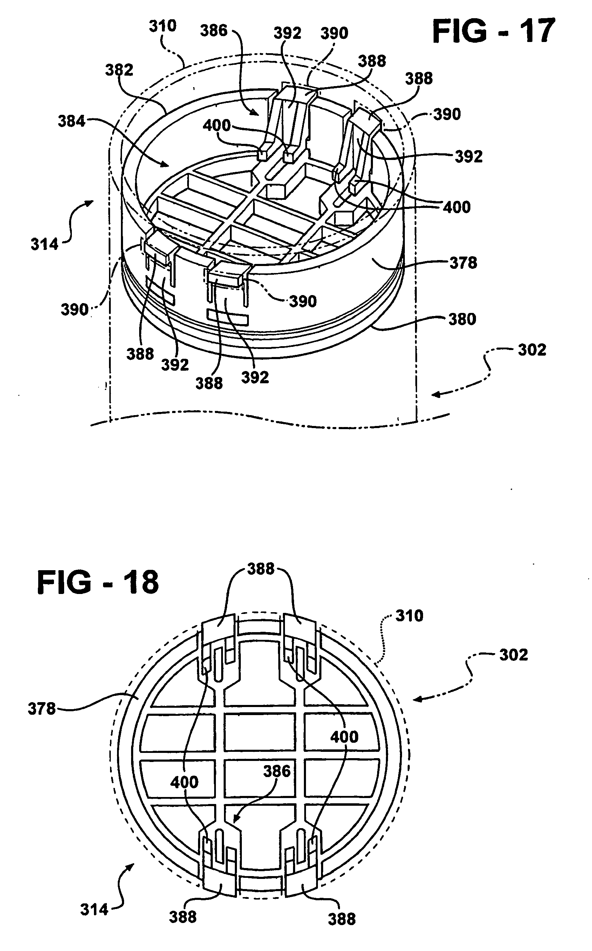

Referring to FIGS. 17-18, an alternative locking member **386** is shown. The alternative locking member **386** includes locking tabs **388** that are biased radially outwardly from the longitudinal axis **L** of the cylinder **302** to engage the slots **390** in the cylinder wall **310.** In this embodiment, four slots **390** are defined in the cylinder wall **310** to receive the locking tabs **388.** The resilient portion **392** is further defined as a resilient base **392** resiliently supporting each of the locking tabs **388** on the piston **314** with each of the locking tabs **388** being radially biased outwardly from the skirt **378** of the piston **314** to engage the slots **390** in the cylinder wall **310.** The release buttons **400** are further defined as fingers **400** extending radially inwardly toward the longitudinal axis **L** of the cylinder **302** with the fingers **400** being engageable to urge the locking tabs **388** radially inwardly and withdraw the locking tabs **388** from the slots **390** in the cylinder wall **310** to release the piston **314** from the locked position.

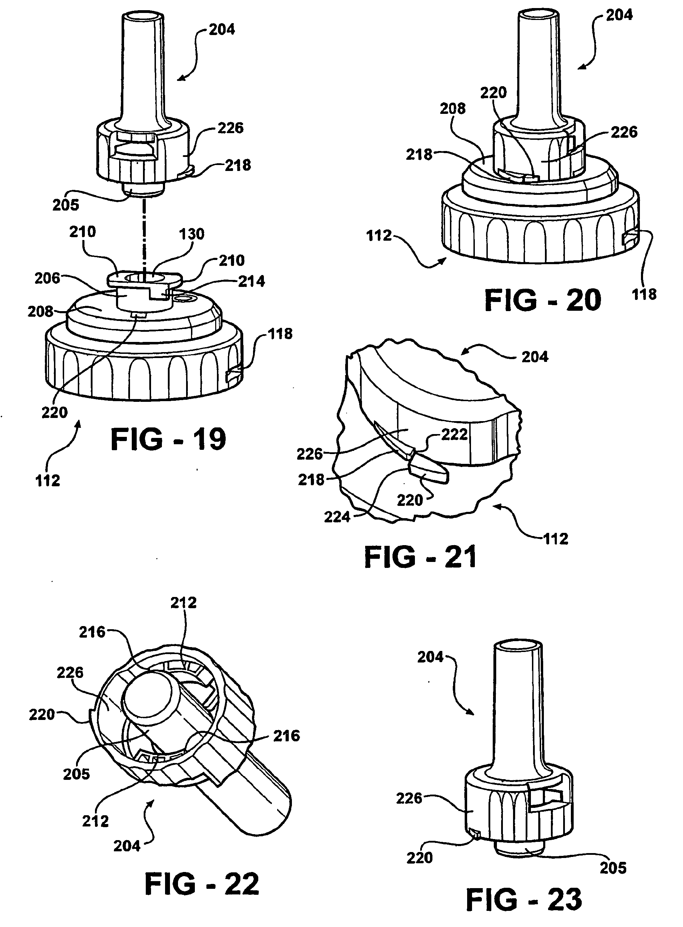

Referring to FIGS. 19-23, once the bone cement is mixed, and the mixing shaft **150** is withdrawn from the cap **112,** the nozzle **204** is positioned on the cap **112.** In the disclosed embodiment, the nozzle **204** is set in place by pushing a hollow shaft **205** of the nozzle **204** down into the orifice **130** of the cap **112** and then twisting the nozzle **204** slightly, about one-quarter turn. The nozzle **204** is attached to the cap **112** to prepare the mixing cartridge **100** for placement into the delivery gun **500.**

The cap **112** has a nipple **206** protruding from an outer surface **208** thereof. The nipple **206** has tabs **210,** which engage detent members **212** in the nozzle **204.** After the nozzle **204** is fully rotated into position, the tabs **210** fully engage the detent members **212** while being positioned proximal to the detent members **212** to secure the nozzle **204** in place. A stop **214** on the cap **112,** best shown in FIG. 19, prevents the nozzle **204** from rotating freely in the clockwise direction after the tabs **210** have engaged the detent members **212.** The stop **214** extends downwardly from one of the tabs **210** to abut a side surface **216** of one of the detent members **212** to prevent further clockwise rotation.

The nozzle **204** and cap **112** have first **218** and second **220** locking protrusions. The first locking protrusion **218** acts as a detent and slides over the second locking protrusion **220** to a locked position as illustrated in FIG. 21. In this position, rear flat surfaces **222, 224** of the locking protrusions **218, 220,** abut one another to prevent the nozzle **204** from being turned in the opposite direction, thereby preventing removal of the nozzle **204** from the cap **112.** The nozzle **204** can be removed by deflecting an outer skirt **226** of the nozzle **204** and rotating the nozzle **204** counterclockwise thereby disengaging the locking protrusions **218, 220.** Both the nozzle **204** and cap **112** are formed from plastic, which facilitates the detent-like locking and unlocking of the nozzle **204** to the cap **112.**

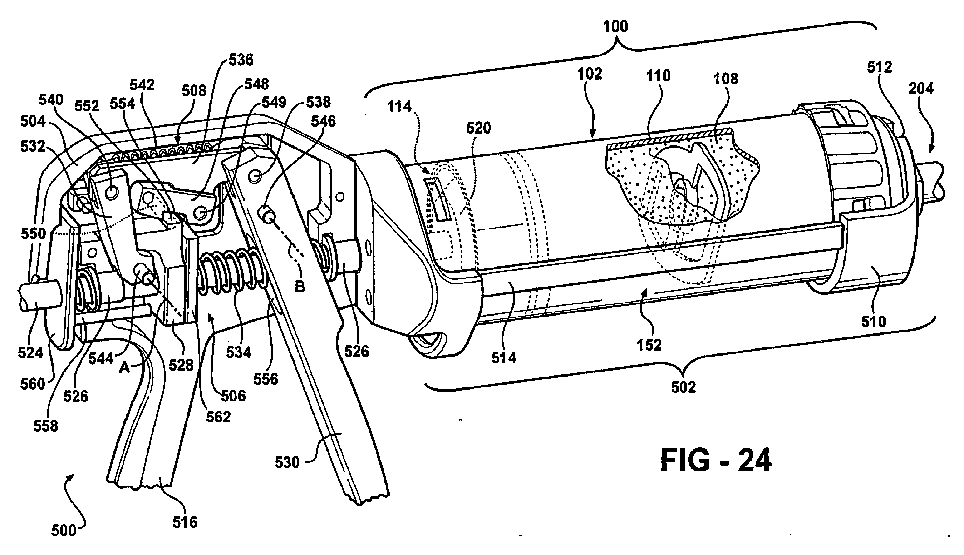

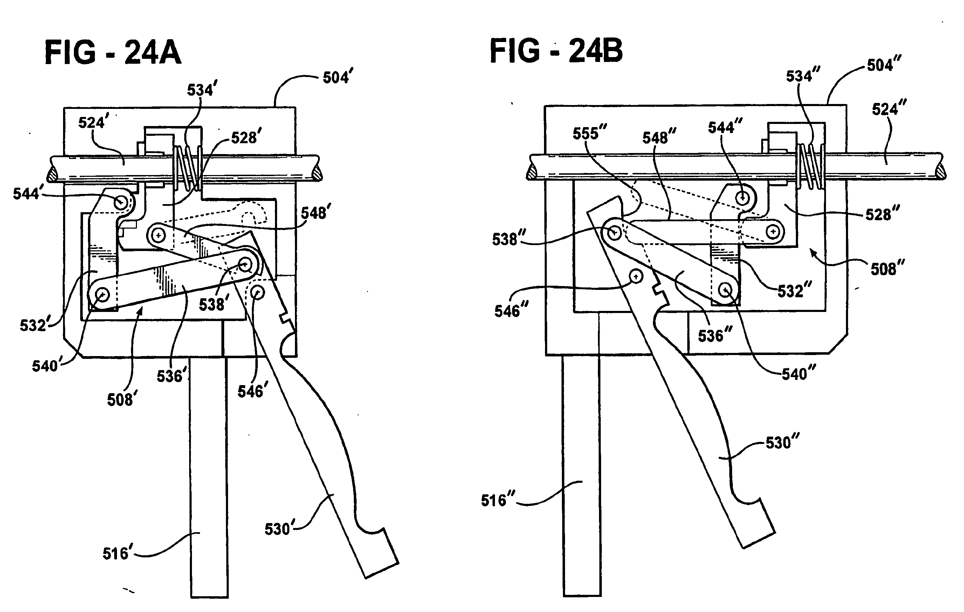

With the nozzle **204** in place, the mixing cartridge **100** is ready to be placed within the delivery gun **500.** Referring to FIG. 24, the delivery gun **500** of the present invention includes a cradle **502** for supporting the mixing cartridge **100** and a casing **504** fixed to the cradle **502** for supporting a drive mechanism **506,** a linkage system **508,** and corresponding components. The cradle **502** includes an endplate **510,** which has an opening **512** for receipt of the nozzle **204.** The endplate **510** holds the mixing cartridge **100** in position in the cradle **502.** In the preferred embodiment, the casing **504** and the endplate **510** are connected by two connecting bars **514** (one on each side of the mixing cartridge **100)** to reduce the weight of the delivery gun **500.** A handle **516** is integrally formed with the casing **504** to maneuver the delivery gun **500** during use.

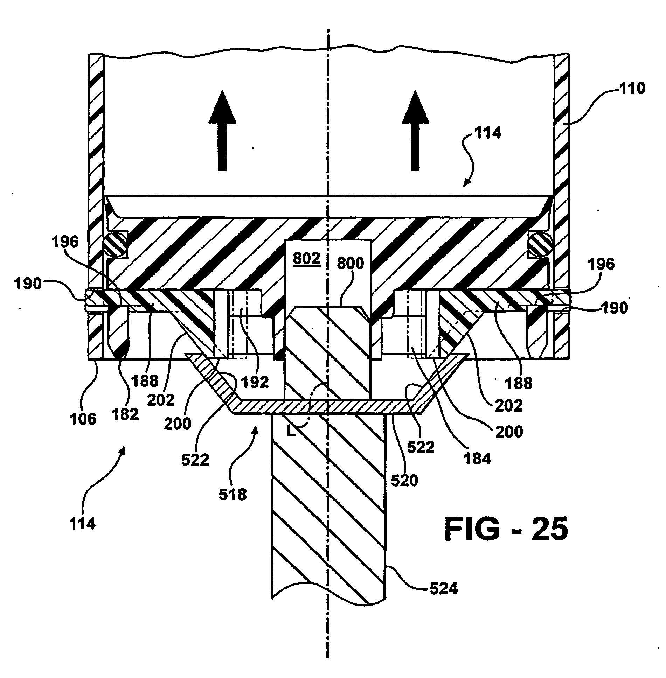

To dispense the bone cement from the mixing cartridge **100,** the piston **114** must first be released from the locked position. Referring to FIG. 25, this is accomplished using a release mechanism **518** integrated into the delivery gun **500.** Once the mixing cartridge **100** is in place in the cradle **502,** a ram disk **520** protrudes into the cavity **184** in the distal end **182** of the piston **114.** The release mechanism **518** is integrated into the ram disk **520.** The release mechanism **518** includes a bearing surface **522** forming an acute angle with the longitudinal axis **L** for catching the release buttons **200** to cam the release buttons **200** radially inwardly. More specifically, the cam surfaces **202** of the release buttons **200** slide along the bearing surface **522,** while being cammed radially inwardly. This action pulls the locking tabs **188** radially inwardly to withdraw the locking tabs **188** from the slots **190** in the cylinder wall **110** and release the piston **114** from the locked position (when the alternative piston **314** is used, the ram disk has a flat bearing surface that axially presses the fingers **400** proximally to bend each resilient base **392** inwardly and urge the locking tabs **388** radially inward). A centering pin **800** can be used to center the ram disk **520** in a centering cavity **802** of the piston **114** to facilitate the release of the piston **114** from the locked position.

Referring back to FIG. 24, once the piston **114** is released, the piston **114** can be driven through the mixing chamber **108** by the drive mechanism **506** to force the bone cement from the nozzle **204.** The drive mechanism **506** includes a drive rod **524** movably supported by bushings **526** in the casing **504.** The ram disk **520** is fixed to the drive rod **524.** The drive mechanism **506** further includes a first gripper plate **528** responsive to movement of the linkage system **508** upon actuation of a trigger **530.** The first gripper plate **528** defines an aperture surrounding the drive rod **524.** The first gripper plate **528** frictionally engages the drive rod **524** to advance the drive rod **524.** The first gripper plate **528** is urged forward while in frictional contact with the drive rod **524** by the linkage system **508** when the trigger **530** is actuated. The first gripper plate **528** thereby advances the drive rod **524** and ram disk **520** relative to the casing **504** to drive the piston **114** and force the bone cement from the mixing cartridge **100.** The trigger **530** is pivotally supported by the casing **504** and operatively connected to the drive mechanism **506** to advance the drive mechanism **506** upon actuation of the trigger **530.**

The linkage system **508** includes a first link **532,** which is pivotally mounted to the casing **504** about a pivot axis **A** adjacent to the first gripper plate **528.** The first link **532** is adapted to engage the first gripper plate **528** when the first link **532** pivots about the pivot axis **A.** A second link **536** pivotally interconnects the trigger **530** to the first link **532** via support pins **538, 540.** The links **532, 536** and trigger **530** are interconnected to move in unison upon rotation of the trigger **530** about a second pivot axis **B.** When the trigger **530** is pulled, the second link **536** rotates the first link **532** about the pivot axis **A,** which engages the first gripper plate **528** and urges the first gripper plate **528** forward while the first gripper plate **528** is in frictional engagement with the drive rod **524** thereby advancing the drive rod **524.** A return spring **542** returns the links **532, 536** and the trigger **530** to an initial position upon release of the trigger **530.** At the same time, a first spring **534** momentarily disengages the first gripper plate **528** from the drive rod **524** to slide the first gripper plate **528** back to an initial position to await the next pull of the trigger **530.** The casing **504** pivotally supports the first link **532** and the trigger **530** about the pivot axes **A** and **B** via support pins **544, 546.**

A speed-changing link **548** is pivotally connected to the second link **536** about a support pin **549.** The speed-changing link **548** selectively pivots into and out from engagement with the first gripper plate **528** by way of a switch **550.** The speed-changing link **548** pivots between a high-speed position and a low-speed position about the support pin **549** (the low-speed position is shown in FIG. 24). The high-speed position corresponds to faster advancement of the drive rod **524** at a lower force. This allows the user to quickly advance the drive rod **524** to drive the piston **114** and dispense high volumes of bone cement at low pressure. The low-speed position corresponds to slower advancement of the drive rod **524** at a higher force, which exerts more force on the piston **114** to pressurize the bone cement

The first gripper plate **528** and the speed-changing link **548** have complementary first and second coupling devices **552, 554** used to couple the first gripper plate **528** with the speed-changing link **548** in the high-speed position. More specifically, in the embodiment of FIG. 24, the first gripper plate **528** has a shoulder **552** that is received within a channel **554** on the speed-changing link **548.** The speed-changing link **548** engages the shoulder **552** in the high-speed position. In the high-speed position, a user's gripping force is transmitted through the trigger **530** to the second link **536** and the speed-changing link **548** to engage the first gripper plate **528** and advance the drive rod **524.** The speed-changing link **548** is isolated from the first gripper plate **528** in the low-speed position. The low-speed position corresponds to the speed-changing link **548** being switched or disconnected from the shoulder **552.** In the low-speed position, the user's gripping force is transmitted through the trigger **530** to both the first **532** and second **536** links to engage the first gripper plate **528** and advance the drive rod **524.** This results in slower advancement of the drive rod **524,** but at a much higher mechanical advantage than the high-speed position. As a result, the user can better pressurize the bone cement during injection.

The pivot axes **A** and **B** and the links **532, 536, 548** are positioned above the drive rod **524,** while the trigger **530** extends below the drive rod **524.** A channel **556** defined in the trigger **530** facilitates this configuration. There are several advantages to this configuration. Moving the second pivot axis **B** away from a user's hand results in better usage of the stronger index and ring fingers by allowing those fingers more travel distance as the trigger **530** is actuated. This configuration also allows the handle **516** to be closer to the drive rod **524,** which is believed to reduce wrist strain when the user pushes the delivery gun **500** forward during cement pressurization. Another benefit is that it allows for a more streamlined casing design and better weight distribution.

In one configuration, shown in FIG. 24, a secondary gripper plate **562** is mounted about the drive rod **524** adjacent to the first gripper plate **528.** The addition of one or more secondary gripper plates **562** to the first gripper plate **528** adds strength to the delivery gun **500** while still permitting proper operation. By using two or more gripper plates **528, 562,** increased frictional contact with the drive rod **524** is obtained without adversely affecting performance.

A release pin **558** disengages the gripper plates **528, 562** to allow a user to freely move the drive rod **524** by hand. The release pin **558** is connected to a retainer plate **560** and is adapted to engage the first gripper plate **528.** When the retainer plate **560** is pushed by the user, the release pin **558** engages the first gripper plate **528** which forces the first gripper plate **528** to tilt back against the bias of the first spring **534** thus releasing the drive rod **524.** Any secondary gripper plates **562** follow. As should be appreciated, pushing the retainer plate **560** also pivots the retainer plate **560** releasing its engagement with the drive rod **524.** With both the retainer plate **560** and the gripper plates **528, 562** released, the drive rod **524** is free to move. This allows the user to manually move the drive rod **524** with respect to the casing **504.**

The delivery gun **500** is unique among bone cement guns with a friction-plate mechanism in the way that it handles wear and deformation of the gripper plates **528, 562.** In the disclosed embodiments, the gripper plates **528, 562** are tilted by the first spring **534** into frictional contact with the drive rod **524.** Regardless of the amount of wear or deformation of the gripper plates **528, 562** or the drive rod **524,** the gripper plates **528, 562** require no further tilting to engage the drive rod **524** upon actuation of the trigger **530.** Thus, advancement of the drive rod **524** is produced over the entire actuation of the trigger **530** and efficiency is maintained throughout the life of the delivery gun **500.**

Referring to FIGS. 24A and 24B, alternatives of the linkage system **508'** and **508"** are shown. These alternatives are represented with similar numerals to the embodiment of FIG. 24 to indicate like parts. FIG. 24A illustrates a configuration of the linkage system **508'** in which the linkage system **508'** lies beneath the drive rod **524'.** Furthermore, the speed-changing link **548'** in this embodiment is pivotally connected to the first gripper plate **528'** and includes a hook-shaped end to engage the support pin **538'** in the high-speed position and disengage the support pin **538'** in the low-speed position. FIG. 24B illustrates a configuration of the linkage system **508"** in which the first gripper plate **528"** is pushed by the linkage system **508",** as opposed to being pulled by the linkage system **508** and **508'** in FIGS. 24 and 24A. Here, the speed-changing link **548"** is pivotally connected to the first gripper plate **528"** to pivot into engagement with a notch 555" defined in the trigger **530"** in the high-speed position and out from engagement with the notch **555"** in the low-speed position. These alternatives of the linkage system **508'** and **508**" illustrate the flexibility of design, e.g., the selection of mechanical advantage, provided by the linkage system.

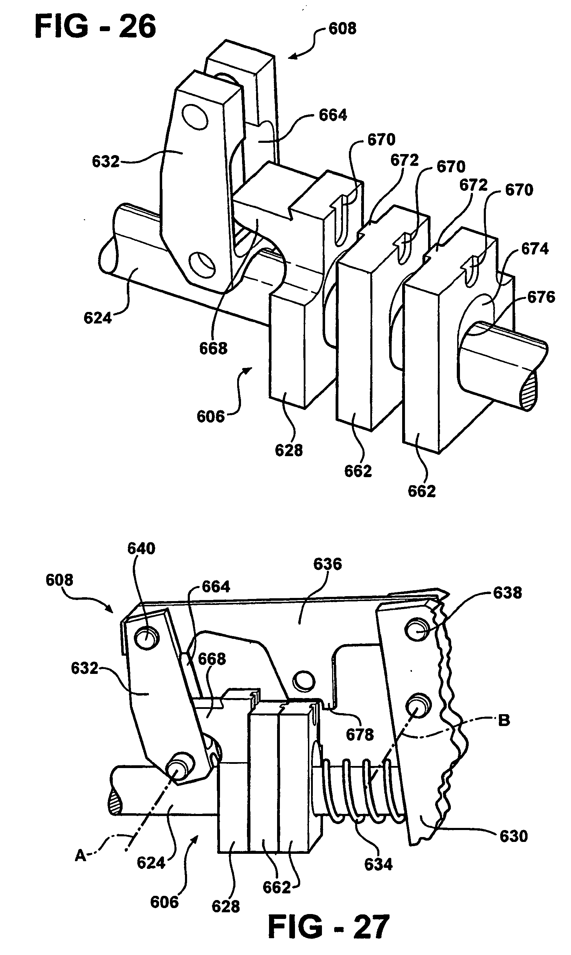

Referring to FIGS. 26-27, alternatives of the drive mechanism **606** and linkage system **608** are shown (only a portion of the drive, mechanism **606** and linkage system **608** is shown for illustrative purposes). In this configuration, the linkage system **608** comprises the same components as previously described with an improved first link **632** and gripper plates **628, 662.** In this embodiment, a plurality of secondary gripper plates **662** are aligned along the drive rod **624** next to the first gripper plate **628.** The first link **632** defines a female recess **664** and the first gripper plate **628** includes a male member **668** for mating engagement with the female recess **664.** The secondary gripper plates **662** are aligned relative to the first gripper plate **628** via mating notches **670** and pegs **672** formed therein. The notches **670** and pegs **672** assume the same shape to mate with one another and maintain alignment. This arrangement minimizes alignment changes that may cause slipping or uneven wear. The arrangement also reduces contact between the gripper plates **628, 662** and an interior wall of the casing **504.** The gripper plates **628, 662** are shown spaced in FIG. 26 for illustration only. In practice, the gripper plates **628, 662** abut one another, as shown in FIG. 27.

In this configuration each of the gripper plates **628, 662** also defines a pair of semi-spherical grooves **674.** In FIG. 26, only the first of the pair of grooves **674** are shown in each of the gripper plates **628, 662.** The other of the pair of grooves **674** is located in a rear surface of each of the gripper plates **628, 662,** cater-cornered from the first of the pair of grooves **674.** These grooves **674** increase the frictional contact with the drive rod **624.** When the gripper plates **628, 662** are urged forward while in frictional engagement with the drive rod **624** by the first link **632,** a substantial portion of a rim **676** defined by each of the grooves **674** frictionally contacts the drive rod **624.**

Referring to FIG. 27, autoclave sterilization of the delivery gun **500** can create a tendency for the gripper plates **628, 662** to adhere to the drive rod **624** beyond their initial positions when the trigger **630** is released. In this situation the first spring **634** cannot produce enough force to disengage the gripper plates **628, 662** from the drive rod **624,** and the gripper plates **628, 662** do not return to their initial positions. FIG. 27 shows a way to prevent this condition. A striker **678,** in the form of a downwardly protruding portion of the second link **636,** closely follows one of the gripper plates **628, 662** during actuation of the trigger **630.** In the event that any of the gripper plates **628, 662** do not properly disengage the drive rod **624** upon release of the trigger **630,** the striker **678** will contact the notch **670** in the closest gripper plate **628, 662** and dislodge the gripper plate **628, 662** from the drive rod **624.** The first spring **634** can then properly return the gripper plates **628, 662** to their initial positions.

A coating has been added to an exterior of each of the gripper plates **528, 562, 628, 662** in FIGS. 24 and 26-27. The coating increases lubricity and corrosion resistance. This facilitates sliding between the gripper plates **528, 562, 628, 662** as they engage the drive rod **524, 624.** The coating also reduces corrosion due to autoclave sterilization that may cause the gripper plates **528, 562, 628, 662** to adhere to one another and prevent proper engagement with the drive rod **524, 624.** The coating used may be Electroless-Nickel with polytetrafluoroethylene (PTFE) or other like coatings possessing the same or similar properties.

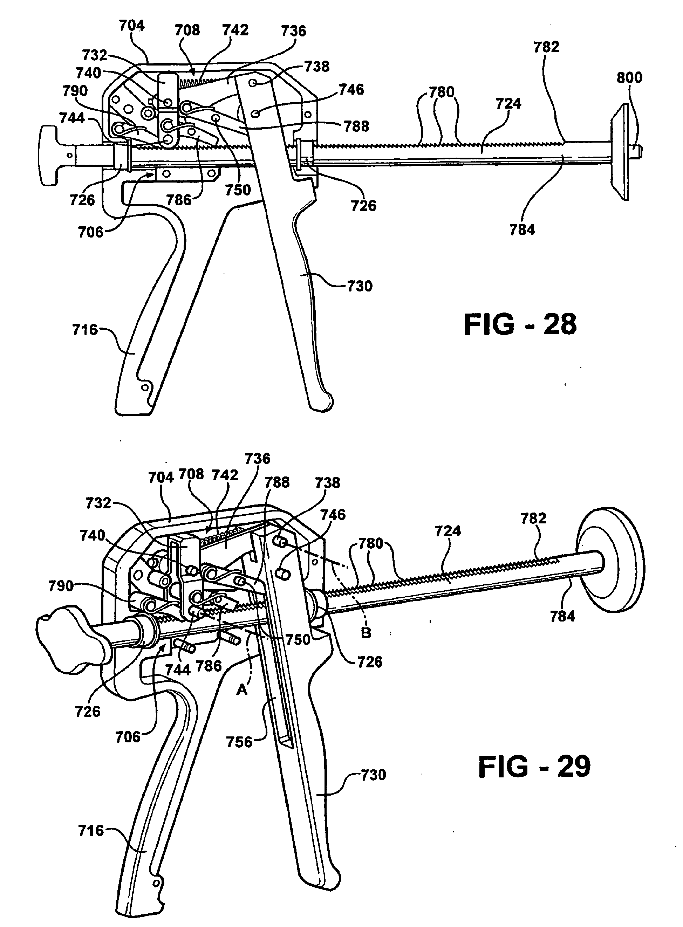

Referring to FIGS. 28-31, alternatives of the drive mechanism **706** and linkage system **708** are shown. This configuration also provides selective high-speed and low-speed advancement of the drive rod **724.** This alternative drive mechanism **706** eliminates the gripper plate by providing teeth **780** on the drive rod **724.** A cross-section of the drive rod **724** shows the teeth **780** on a flat upper surface **782,** while a lower surface **784** is smooth and round. The first link **732,** which in previous embodiments urged the first gripper plate **528, 628** forward with the drive rod **524, 624,** now pivotally supports a first pawl member **786.** The first pawl member **786** is spring-biased into engagement with the teeth **780.**

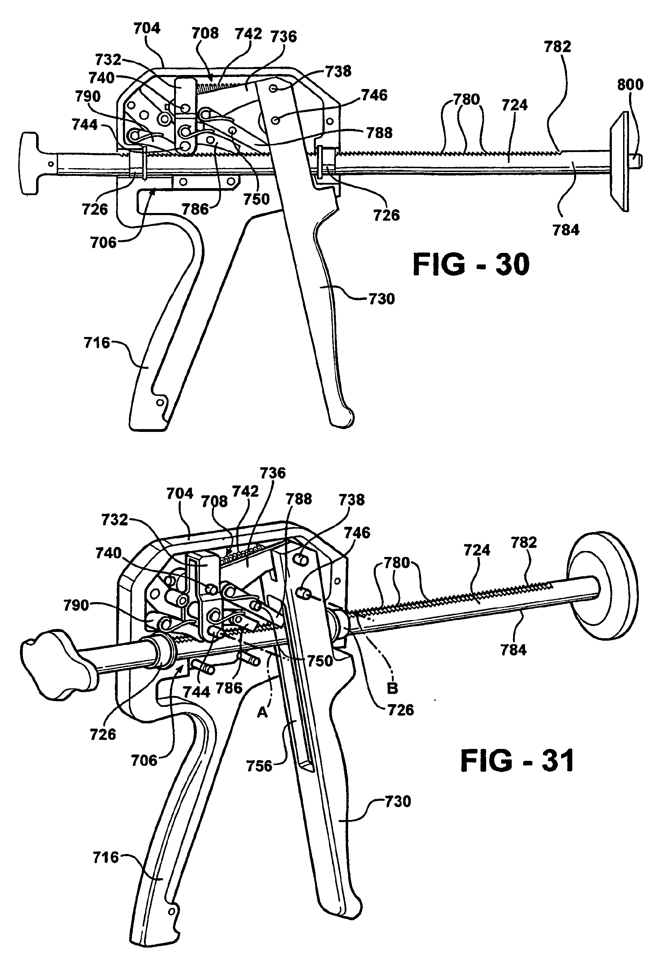

A second pawl member **788** is pivotally supported by the second link **736.** The second pawl member **788** is pivotable between a high-speed position in which the second pawl member **788** is spring-biased into engagement with the teeth **780** to advance the drive rod **724,** and a low-speed position in which the second pawl member **788** is disengaged and isolated from the teeth **780.** In the low-speed position, the first pawl member **786** advances the drive rod **724.** The low-speed position is illustrated in FIGS. 28-29. In the high-speed position, with the second pawl member **788** engaging the teeth **780,** the first pawl member **786** remains in engagement with the teeth **780,** but only ratchets along the teeth **780** as the second pawl member **788** advances the drive rod **724.** The high-speed position is illustrated in FIGS. 30-31.. The principle of increasing mechanical advantage in the low-speed position relative to the high-speed position also applies in this embodiment.

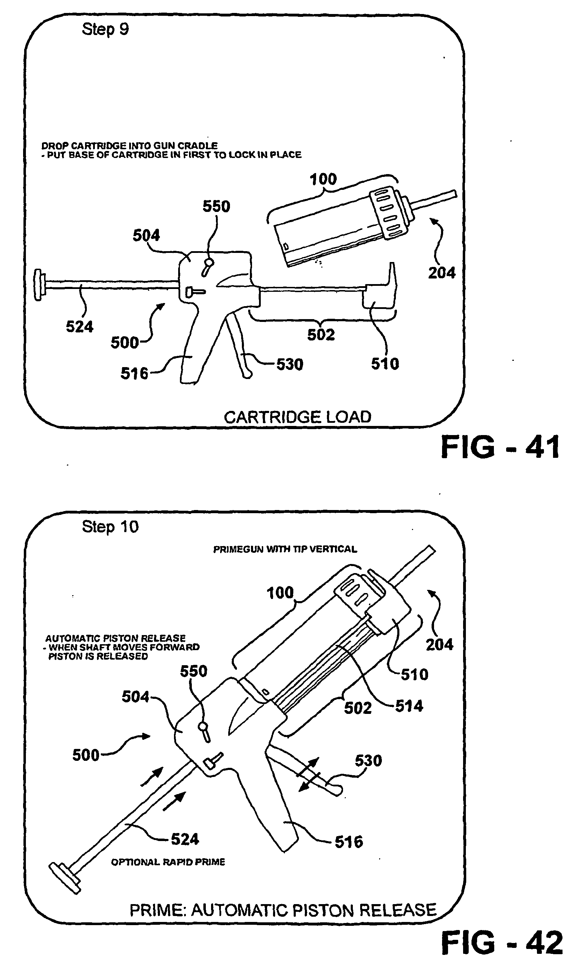

The switch **750** is used to pivot the second pawl member **788** out from engagement with the teeth **780** of the drive rod **724** in the low-speed position (see FIGS. 28-29) and into engagement with the teeth **780** in the high-speed position (see FIGS. 30-31). A switch similar to that shown in United States Patent No. 5,431,654 to Nic, herein incorporated by reference, can be used for this purpose. The switch **750** extends through the casing **704** and terminates in a button that is manipulated by a user to move the second pawl member **788** between the high-speed and low-speed positions (see briefly FIGS. 41-42). This also applies to the switch **550** used to move the speed-changing link **548** in previous embodiments.

In this configuration, the retainer plate **560** can be removed. In its place, a spring-biased non-return pawl member **790** retains the drive rod **724** in position upon advancement. The drive rod **724** can be freely moved in the casing **704** by rotating the drive rod **724** one hundred and eighty degrees such that the pawl members **786, 788, 790** are out of engagement with the teeth **780.** Upon such rotation, the pawl members **786, 788, 790** ride on the smooth lower surface **784** of the drive rod **724** allowing the user to freely pull the drive rod **724** relative to the casing **704.** This is generally disclosed in the '654 patent to Nic.

Each of the pawl members **786, 788, 790** are pivotally supported by pins. Springs, such as those shown in the `654 patent to Nic, bias the pawl members into engagement with the teeth **780** on the drive rod **724** (except when the switch **750** acts against the bias of the spring in the low-speed position to disengage the second pawl member **788** from the teeth **780).**

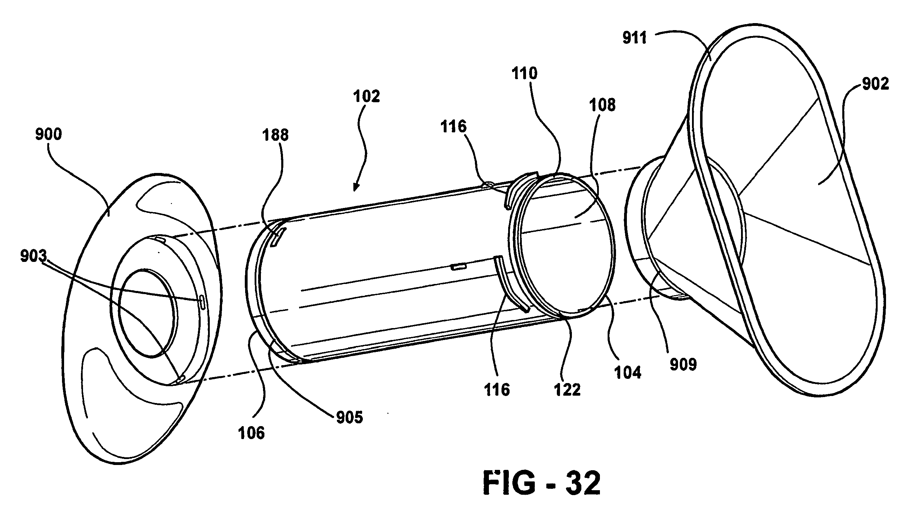

Mixing and delivery of the bone cement will now be described with reference to FIGS. 32-42. Referring first to FIG. 32, a bone cement loading system is shown. The bone cement loading system comprises a base **900** supporting the cylinder **102** while loading the liquid and powder components of the bone cement into the mixing chamber **108.** The base includes a cavity for receiving the distal end **106** of the cylinder **102.** Detents **903** are formed in the cavity. A groove **905** is defined in an outer surface of the cylinder **102** to receive the detents **903** and facilitate a snug fit between the base **900** and the cylinder **102.** It should be appreciated that the detents **903** could be formed on the cylinder **102** with the groove **905** defined in the base **900.** The distal end **106** of the cylinder **102** may also be press fit into the base **900.** The base **900** is oblong and oval in shape to fully support the cylinder **102** on a work surface, while the cavity is circular in shape to fit the circular shaped cylinder **102.** A funnel **902** couples to the cylinder **102** to channel the powder into the cylinder **102** during loading. The funnel **902** includes a proximal end **911** having an oblong oval-shaped periphery to facilitate the loading of the powder into the mixing chamber **108** and a distal end **909** having a circular periphery to snugly fit inside the proximal end **104** of the cylinder **102.**

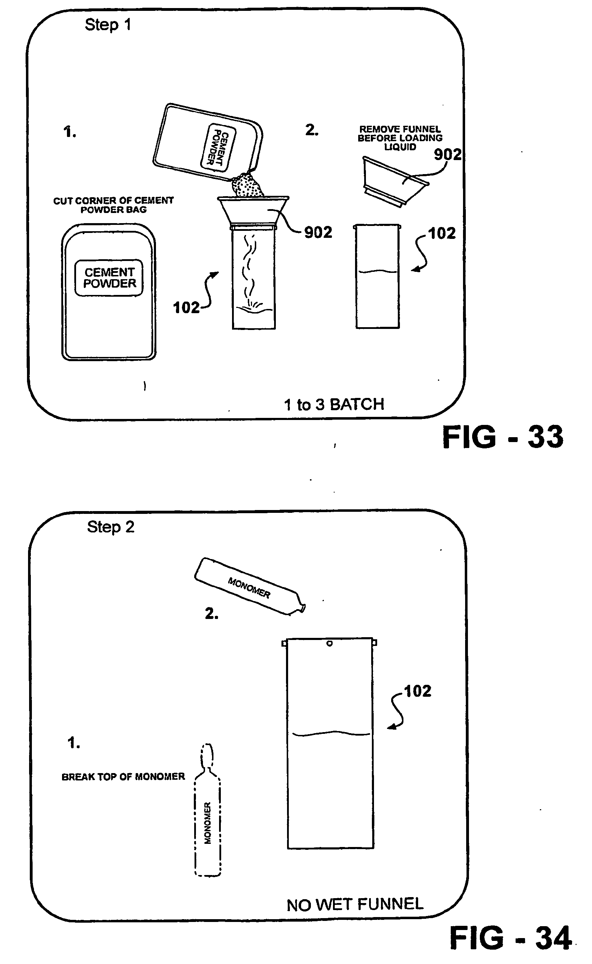

FIGS 33-42 illustrate ten steps for preparing and injecting the bone cement. The mixing cartridge 100, delivery gun 500, and other components are generically shown in each step for illustrative purposes only.

In STEP 1, shown in FIG. 33, the funnel 902 is coupled to the cylinder 102 and the powder is poured into the mixing chamber 108.

In STEP 2, shown in FIG. 34, after the powder is poured into the mixing chamber 108, the funnel 902 is removed, and the liquid component, e.g., liquid monomer, of the bone cement is added. In this manner, the present disclosure avoids wetting of the funnel 902 and the associated clean-up.

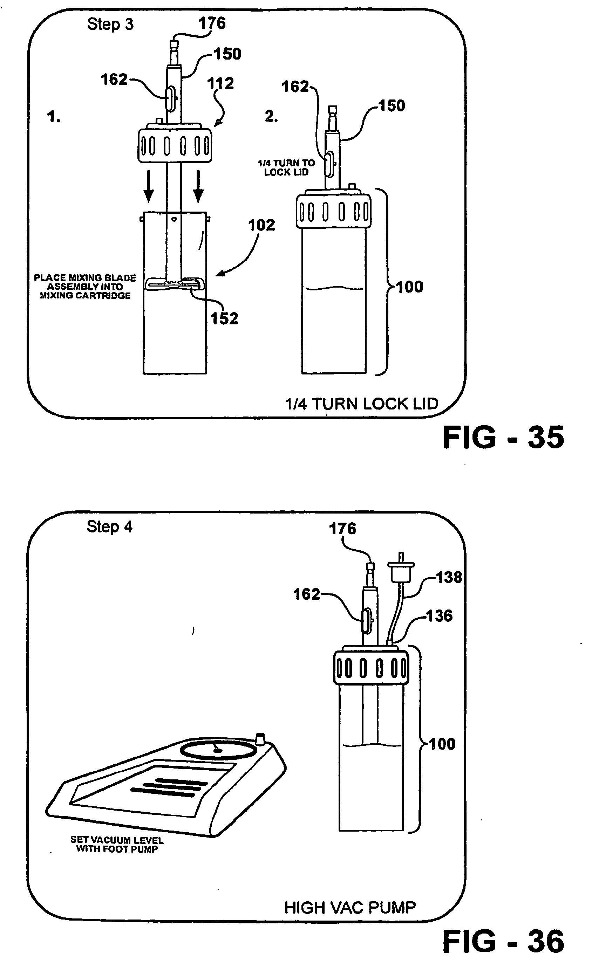

In STEP 3, shown in FIG. 35, the cap **112** with the mixing shaft **150** and blade 152 supported therein is attached to the cylinder **102.**

In STEP 4, shown in FIG. 36, the vacuum line **138** is attached to the vacuum port 136 and a vacuum is drawn in the mixing chamber **108** with the liquid and powder components therein.

In STEP 5, shown in FIG. 37, with the vacuum drawn, the power tool (reamer) is then connected to the mixing shaft **150.**

In STEP 6, shown in FIG. 38, with the vacuum still drawn, the mixing shaft 150 is moved axially with respect to the mixing cartridge **100** and rotated by the power tool. The blade **152** (not shown in FIG. 38) is moved axially the entire extent of the mixing cartridge **100** while rotating to ensure that the liquid and powder components are fully mixed.

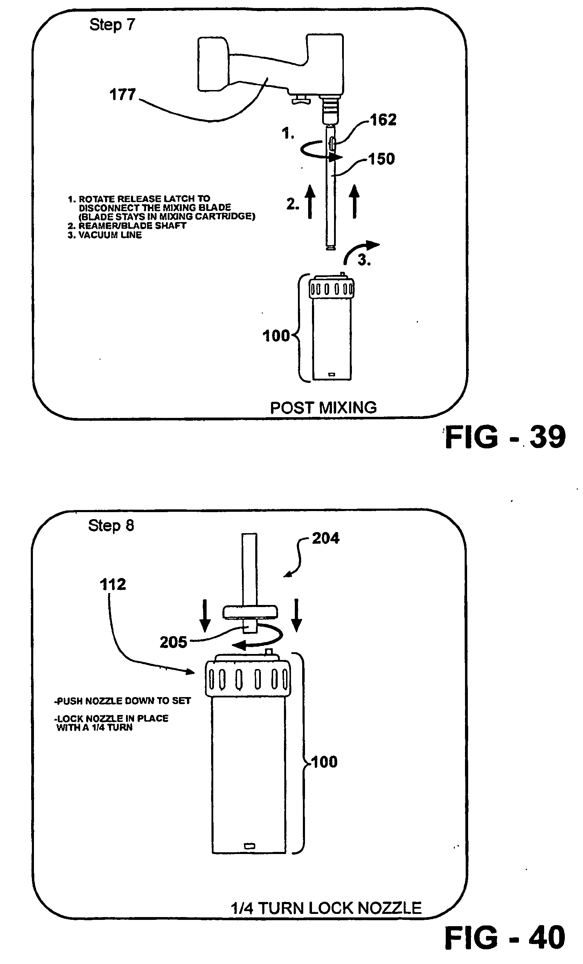

In STEP 7, shown in FIG. 39, once mixed, the release latch **162** is moved to release the blade **152** (not shown in FIG. 39). The blade **152** remains in the mixing chamber **108** once released. The mixing shaft **150** is then removed from the mixing cartridge **100.** Mixing is now complete.

In STEP 8, shown in FIG. 40, the nozzle **204** is pushed down on the cap **112** and rotated into place.

In STEP 9, shown in FIG. 41, the mixing cartridge **100** is positioned in the cradle **502.**

In STEP, shown in FIG. 42, the piston **114** is released from the distal end **106** of the cylinder **102** and the delivery gun **500** is primed and ready to discharge the bone cement from the mixing cartridge **100.**

It will be appreciated that the above description relates to the disclosed embodiments by way of example only. Many apparent variations of the disclosed invention will be know to those of skill in this area and are considered to be within the scope of this invention and are considered to be within the scope of the following claims. Obviously, many modifications and variations of the present invention are possible in light of the above teachings.

## Claims

1. A mixing cartridge (100) adapted for use with a delivery device (500) discharging bone cement from the mixing cartridge (100), said mixing cartridge (100) comprising:
a wall (110, 310) having proximal (104) and distal (106) ends and including a plurality of first connecting portions (190, 390) at said distal end (106);
a piston (114, 314) releasably secured to said wall (110, 310) at said distal end (106) to define a chamber to hold the bone cement;
a vacuum port (136) in communication with said chamber for receiving a conduit to draw suction into said chamber;
a locking member (186, 386) operatively coupled to said piston (114, 314) and including a plurality of second connecting portions (188, 388) each for engaging a respective first connecting portion (190, 390) of said wall (110, 310) to place said piston (114, 314) in a releasably locked position in said distal end (106) of said wall (110, 310); and
**characterized in that**
said first connecting portions are each defined as a slot (190, 390) defined in said wall (110, 310); and
said second connecting portions are each defined as a locking tab (188, 388) protruding into one of said slots (190, 390) in said locked position, wherein the locking tab (188, 388) includes a release member (200, 400) adapted to be engaged by said delivery device (500) so that engagement of said release member (200, 400) by said delivery device (500) withdraws said locking tab (188, 388) from said slot (190, 390) in a radial inward direction away from the wall (110, 310) so as to unlock said piston (114, 314) from said wall (110, 310).

2. The cartridge (100) as set forth in claim 1, wherein said piston (114, 314) includes a proximal end (180, 380) and a distal end (182, 382) with a cavity (184, 384) defined in said distal end (182, 382), said second connecting portions (188, 388) being disposed in said cavity (184, 384).

3. The cartridge (100) as set forth in claim 1 or 2, wherein a resilient portion (192) extends between said locking tabs (188) to bias said locking tabs (188) outwardly toward said slots (190).

4. The cartridge (100) as set forth in claim 3, wherein said resilient portion (192) is integral with said locking tabs (188) and comprises a resilient strip (192) extending between said locking tabs (188).

5. The cartridge (100) as set forth in one of claims 1 to 4, wherein said piston (114) defines a pair of carrier slots (194) for receiving and axially securing said locking tabs (188) when said piston (114) is in said locked position and after said piston (114) is unlocked.

6. The cartridge (100) as set forth in one of claims 1 to 5, wherein said release members (200, 400) are integrally formed with said locking tabs (188, 388).

7. The cartridge (100) as set forth in one of claims 1 to 6, wherein each of said release members (200) includes a cam surface (202) for being engaged by the delivery device (500) to withdraw said locking tabs (188) from said slots (190) in said wall (110) and unlock said piston (114).

8. The cartridge (100) as set forth in one of claims 1 to 7, including a cap (112) connected to said wall (110, 310) adjacent said proximal end (104).

9. The cartridge (100) as set forth in one of claims 1 to 8, including a mixing blade (152) disposed in said chamber (108) for mixing components of the bone cement while drawing suction through said vacuum port (136), said locking member (186, 386) securing said piston (114, 314) in said locked position during mixing.

10. The cartridge (100) as set forth in one of claims 1 to 9, wherein said piston (114, 314) includes a proximal end (180, 380), a distal end (182, 382), and a skirt (178, 378) extending between said ends (180, 182, 380, 382) with a cavity (184) defined radially inwardly of said skirt (178, 378) of said piston (114, 314) adjacent said distal end (182, 382) of said piston (114, 314), wherein said release members (200, 400) are disposed in said cavity (184, 384) such that the delivery device (500) at least partially enters said cavity (184, 384) to engage said release members (200, 400) and unlock said piston (114, 314).

11. A bone cement system for combining liquid and powder components of bone cement and delivering the bone cement to an anatomical site of a patient, said system comprising:
a cartridge (100) as set forth in any of claims 1 to 10,
a delivery device (500, 706) adapted to hold said cartridge (100), the delivery device (500) comprising:
a casing (504, 704), and
a drive mechanism (506, 606, 706) supported by said casing (504) and advanceable relative to said casing (504) to discharge the bone cement mixture from said cartridge (100), wherein said drive mechanism (506, 606, 706) includes a release mechanism (518) for engaging said release members (200, 400) so as to unlock said piston (114, 314) from the wall (110, 310).

12. The bone cement system as set forth in claim 11, wherein the delivery device (500) further comprises
a trigger (530, 730) pivotally supported by said casing (504, 704) and operatively connected to said drive mechanism (506, 706) to actuate said drive mechanism (506, 706) upon actuation of said trigger (530, 730); and
a linkage system (508, 708) operatively interconnecting said trigger (530, 730) with said drive mechanism (506, 730) to actuate said drive mechanism (506, 706), said linkage system (508, 730) comprising a first link (532, 732) pivotally connected to said casing (504, 704) and a second link (536, 736) pivotally interconnecting said first link (532, 732) and said trigger (530, 730) such that actuating said trigger (530, 730) moves said second link (536, 736) and said first link (532, 732) to actuate said drive mechanism (506, 706) and advance said drive mechanism (506, 706) to discharge the bone cement from the cartridge (100).

13. The bone cement system as set forth in claim 12, wherein said drive mechanism (508) further comprises:
a drive rod (524); and
a first gripper plate (528) responsive to actuation of said trigger (530) wherein said first gripper plate (528) surrounds said drive rod (524) and fractionally engages said drive rod (524) to advance said drive rod (524) when said trigger (530) is actuated.

14. The bone cement system as set forth in one of claims 11 to 13, wherein said release members (200) each includes a cam surface (202) engageable by said release mechanism (518) to slide said release members (200) radially inwardly and unlock said piston (114).

15. The bone cement system as set forth in one of claims 11 to 13, wherein said plurality of release members (400) are further defined as a plurality of fingers (400) engageable by said release mechanism (518) to move said plurality of fingers (400) and unlock said piston.

## Patentansprüche

1. Mischkartusche (100), ausgebildet für den Gebrauch mit einem Fördergerät (500), das Knochenzement aus der Mischkartusche (100) entlädt, wobei die Mischkartusche (100) umfasst:
eine Wandung (110, 310) mit einem proximalen (104) und einem distalen (106) Ende sowie mehreren ersten Verbindungselementen (190, 390) am distalen Ende (106);
einen Kolben (114, 314), der lösbar an der Wandung (110, 310) am distalen Ende (106) befestigt ist, um eine Kammer zur Aufnahme des Knochenzements zu bilden;
einen Unterdruckanschluss (136), der mit der Kammer kommuniziert, zur Aufnahme einer Leitung, um Sog an die Kammer anzulegen;
ein Sperrglied (186, 386), das funktional mit dem Kolben (114, 314) verbunden ist und mehrere zweite Verbindungselemente (188, 388) enthält, die jeweils mit einem ersten Verbindungselement (190, 390) der Wandung (110, 310) ineinander greifen, um den Kolben (114, 314) in einer lösbar gesperrten Position am distalen Ende (106) der Wandung (110, 310) zu platzieren,
**dadurch gekennzeichnet, dass**
die ersten Verbindungselemente jeweils als ein Schlitz (190, 390) in der Wandung (110, 310) ausgeführt sind; und
die zweiten Verbindungselemente jeweils als ein Sperrsteg (188, 388) ausgeführt sind, der in der gesperrten Position in einen der Schlitze (190, 390) ragt, wobei der Sperrsteg (188, 388) ein Entriegelungselement (200, 400) enthält, das dazu ausgebildet ist, vom Fördergerät (500) erfasst zu werden, so dass ein Erfassen des Entriegelungselements (200, 400) durch das Fördergerät (500) den Sperrsteg (188, 388) aus dem Schlitz (190, 390) radial nach innen und von der Wandung weg (110, 310) zurückzieht, um so den Kolben (114, 314) von der Wandung (110, 310) zu entsperren.

2. Kartusche (100) nach Anspruch 1, wobei der Kolben (114, 314) ein proximales Ende (180, 380) und ein distales Ende (182, 382) besitzt, wobei im distalen Ende (182, 382) ein Hohlraum (184, 384) ausgeführt ist und die zweiten Verbindungselemente (188, 388) in dem Hohlraum (184, 384) angeordnet sind.

3. Kartusche (100) nach Anspruch 1 oder 2, wobei sich ein Federglied (192) zwischen den Sperrstegen (188) erstreckt, um die Sperrstege (188) nach außen in Richtung der Schlitze (190) vorzuspannen.

4. Kartusche (100) nach Anspruch 3, wobei das Federglied (192) Bestandteil der Sperrstege (188) ist und einen Federstreifen (192) umfasst, der sich zwischen den Sperrstegen (188) erstreckt.

5. Kartusche (100) nach einem der Ansprüche 1 bis 4, wobei der Kolben (114) ein Paar von Trägerschlitzen (194) aufweist zur Aufnahme und axialen Sicherung der Sperrstege (188), wenn sich der Kolben (114) in der gesperrten Position befindet und auch nachdem der Kolben (114) entriegelt worden ist.

6. Kartusche (100) nach einem der Ansprüche 1 bis 5, wobei die Entriegelungselemente (200, 400) als Bestandteile der Sperrstege (188, 388) ausgebildet sind.

7. Kartusche (100) nach einem der Ansprüche 1 bis 6, wobei jedes Entriegelungselement (200) eine Nockenfläche (202) enthält, die vom Fördergerät (500) erfasst werden kann, um die Sperrstege (188) aus den Schlitzen (190) in der Wandung (110) zurückzuziehen und den Kolben (114) zu entsperren.

8. Kartusche (100) nach einem der Ansprüche 1 bis 7, außerdem enthaltend eine Kappe (112), die mit der Wandung (110, 310) benachbart zum proximalen Ende (104) verbunden ist.

9. Kartusche (100) nach einem der Ansprüche 1 bis 8, außerdem enthaltend ein Mischblatt (152), das in der Kammer (108) angeordnet ist, um Komponenten des Knochenzements zu vermischen, während Sog durch den Unterdruckanschluss (136) anliegt, wobei das Sperrglied (186, 386) den Kolben (114, 314) während des Mischens in der gesperrten Position hält.

10. Kartusche (100) nach einem der Ansprüche 1 bis 9, wobei der Kolben (114, 314) ein proximales Ende (180, 380), ein distales Ende (182, 382) und einen Mantel (178, 378) umfasst, der sich zwischen den Enden (180, 182, 380, 382) erstreckt, mit einem Hohlraum (184), der radial innerhalb des Mantels (178, 378) des Kolbens (114, 314) am distalen Ende (182, 382) des Kolbens (114, 314) ausgeführt ist, wobei die Entriegelungselemente (200, 400) in dem Hohlraum (184, 384) so angeordnet sind, dass das Fördergerät (500) wenigstens teilweise in den Hohlraum (184, 384) hineintritt, um die Entriegelungselemente (200, 400) zu erfassen und den Kolben (114, 314) zu entsperren.

11. Knochenzementsystem zum Vermischen von flüssigen und Pulverkomponenten von Knochenzement sowie zum Fördern des Knochenzements an eine anatomische Stelle des Patienten, umfassend:
eine Kartusche (100) nach einem der Ansprüche 1 bis 10,
ein Fördergerät (500, 706), dazu ausgebildet, die Kartusche (100) zu halten, wobei das Fördergerät (500) umfasst:
ein Gehäuse (504, 704), und
einen Betätigungsmechanismus (506, 606, 706), der vom Gehäuse (504) gehalten wird und sich relativ zum Gehäuse (504) vorwärts bewegen lässt, um die Knochenzementmischung aus der Kartusche (100) zu entladen, wobei der Betätigungsmechanismus (506, 606, 706) einen Entriegelungsmechanismus (518) enthält, um die Entriegelungselemente (200, 400) zu erfassen, sodass der Kolben (114, 314) von der Wandung (110, 310) entsperrt wird.

12. Knochenzementsystem nach Anspruch 11, wobei das Fördergerät außerdem umfasst:
einen Auslöser (530, 730), der schwenkbar vom Gehäuse (504, 704) gehalten ist und der funktional mit dem Betätigungsmechanismus (506, 706) verbunden ist, um den Betätigungsmechanismus (506, 706) bei Betätigung des Auslösers (530, 730) zu betätigen; und
ein Verbindungssystem (508, 708), das den Auslöser (530, 730) funktional mit dem Betätigungsmechanismus (506, 706) verbindet, um den Betätigungsmechanismus (506, 706) zu betätigen, wobei das Verbindungssystem (508, 708) eine erste Verbindung (532, 732) umfasst, die schwenkbar mit dem Gehäuse (504, 704) verbunden ist, und eine zweite Verbindung (536, 736), die die erste Verbindung (532, 732) und den Auslöser (530, 730) schwenkbar so verbindet, dass ein Betätigen des Auslösers (530, 730) die zweite Verbindung (536, 736) und die erste Verbindung (532, 732) bewegt, um den Betätigungsmechanismus (506, 706) zu betätigen und den Betätigungsmechanismus (506, 706) vorwärts zu bewegen, um Knochenzement aus der Kartusche (100) zu entladen.

13. Knochenzementsystem nach Anspruch 12, wobei der Betätigungsmechanismus (508) außerdem umfasst:
eine Betätigungsstange (524); und
eine erste Greiferplatte (528), die auf eine Betätigung des Auslösers (530) reagiert, wobei die erste Greiferplatte (528) die Betätigungsstange (524) umgibt und die Betätigungsstange (524) reibschlüssig erfasst, um die Betätigungsstange (524) vorwärts zu bewegen, wenn der Auslöser (530) betätigt wird.

14. Knochenzementsystem nach einem der Ansprüche 11 bis 13, wobei die Entriegelungselemente (200) jeweils eine Nockenfläche (202) aufweisen, die vom Entriegelungsmechanismus (518) erfasst werden kann, um die Entriegelungselemente (200) radial nach innen zu verschieben und den Kolben (114) zu entsperren.

15. Knochenzementsystem nach einem der Ansprüche 11 bis 13, wobei die mehreren Entriegelungselemente (400) außerdem als mehrere Finger (400) ausgeführt sind, die vom Entriegelungsmechanismus (518) erfasst werden können, um die mehreren Finger (400) zu bewegen und den Kolben zu entsperren.

## Revendications

1. Cartouche de mélange (100) adaptée à une utilisation avec un dispositif d'administration (500) déchargeant un ciment osseux à partir de la cartouche de mélange (100), ladite cartouche de mélange (100) comprenant :
une paroi (110, 310) ayant des extrémités proximale (104) et distale (106) et incluant une pluralité de premières parties de connexion (190, 390) au niveau de ladite extrémité distale (106) ;
un piston (114, 314) fixé de façon libérable à ladite paroi (110, 310) au niveau de ladite extrémité distale (106) pour définir une chambre pour contenir le ciment osseux ;
un orifice d'aspiration (136) en communication avec ladite chambre pour recevoir un conduit pour créer une aspiration dans ladite chambre ;
un élément de verrouillage (186, 386) opérationnellement couplé audit piston (114, 314) et incluant une pluralité de deuxièmes parties de connexion (188, 388), chacune pour engager une première partie de connexion (190, 390) respective de ladite paroi (110, 310) pour placer ledit piston (114, 314) dans une position verrouillée libérable dans ladite extrémité distale (106) de ladite paroi (110, 310) ; et
**caractérisée en ce que**
lesdites premières parties de connexion sont chacune définies comme une fente (190, 390) définie dans ladite paroi (110, 310) ; et
lesdites deuxièmes parties de connexion sont chacune définies comme un onglet de verrouillage (188, 388) faisant saillie à l'intérieur d'une desdites fentes (190, 390) dans ladite position verrouillée, dans laquelle l'onglet de verrouillage (188, 388) inclut un élément de libération (200, 400) adapté à être engagé par ledit dispositif d'administration (500) de façon à ce que l'engagement dudit élément de libération (200, 400) par ledit dispositif d'administration (500) fasse sortir ledit onglet de verrouillage (188, 388) de ladite fente (190, 390) dans un sens radial vers l'intérieur à l'écart de la paroi (110, 310) de manière à déverrouiller ledit piston (114, 314) de ladite paroi (110, 310).

2. Cartouche (100) selon la revendication 1, dans laquelle ledit piston (114, 314) inclut une extrémité proximale (180, 380) et une extrémité distale (182, 382) avec une cavité (184, 384) définie dans ladite extrémité distale (182, 382), lesdites deuxièmes parties de connexion (188, 388) étant disposées dans ladite cavité (184, 384).

3. Cartouche (100) selon la revendication 1 ou 2, dans laquelle une partie résiliente (192) s'étend entre lesdits onglets de verrouillage (188) pour pousser lesdits onglets de verrouillage (188) vers l'extérieur vers lesdites fentes (190).

4. Cartouche (100) selon la revendication 3, dans laquelle ladite partie résiliente (192) est intégrée avec lesdits onglets de verrouillage (188) et comprend une bande résiliente (192) s'étendant entre lesdits onglets de verrouillage (188).

5. Cartouche (100) selon l'une des revendications 1 à 4, dans laquelle ledit piston (114) définit une paire de fentes support (194) pour recevoir et fixer axialement lesdits onglets de verrouillage (188) lorsque ledit piston (114) est dans ladite position verrouillée et après que ledit piston (114) a été déverrouillé.

6. Cartouche (100) selon l'une des revendications 1 à 5, dans laquelle lesdits éléments de libération (200, 400) sont formés de façon intégrale avec lesdits onglets de verrouillage (188, 388).

7. Cartouche (100) selon l'une des revendications 1 à 6, dans laquelle chacun desdits éléments de libération (200) inclut une surface de came (202) destinée à être engagée par le dispositif d'administration (500) pour faire sortir lesdits onglets de verrouillage (188) desdites fentes (190) dans ladite paroi (110) et déverrouiller ledit piston (114).

8. Cartouche (100) selon l'une des revendications 1 à 7, incluant une coiffe (112) connectée à ladite paroi (110, 310) de façon adjacente à ladite extrémité proximale (104).

9. Cartouche (100) selon l'une des revendications 1 à 8, incluant une lame de mélange (152) disposée dans ladite chambre (108) pour mélanger des composants du ciment osseux tout en créant une aspiration à travers ledit orifice d'aspiration (136), ledit élément de verrouillage (186, 386) fixant ledit piston (114, 314) dans ladite position verrouillée pendant le mélange.

10. Cartouche (100) selon l'une des revendications 1 à 9, dans laquelle ledit piston (114, 314) inclut une extrémité proximale (180, 380), une extrémité distale (182, 382), et une jupe (178, 378) s'étendant entre lesdites extrémités (180, 182, 380, 382) avec une cavité (184) définie radialement vers l'intérieur de ladite jupe (178, 378) dudit piston (114, 314) de façon adjacente à ladite extrémité distale (182, 382) dudit piston (114, 314), dans laquelle lesdits éléments de libération (200, 400) sont disposés dans ladite cavité (184, 384) de façon à ce que le dispositif d'administration (500) pénètre au moins partiellement dans ladite cavité (184, 384) pour engager lesdits éléments de libération (200, 400) et déverrouiller ledit piston (114, 314).

11. Système pour ciment osseux pour combiner des composants liquides et pulvérulents de ciment osseux et administrer le ciment osseux sur un site anatomique d'un patient, ledit système comprenant :
une cartouche (100) selon l'une quelconque des revendications 1 à 10,
un dispositif d'administration (500, 706) adapté à contenir ladite cartouche (100), le dispositif d'administration (500) comprenant :
un boîtier (504, 704), et
un mécanisme d'entraînement (506, 606, 706) supporté par ledit boîtier (504) et pouvant avancer par rapport audit boîtier (504) pour décharger le mélange de ciment osseux hors de ladite cartouche (100), dans lequel ledit mécanisme d'entraînement (506, 606, 706) inclut un mécanisme de libération (518) pour engager lesdits éléments de libération (200, 400) de façon à déverrouiller ledit piston (114, 314) de la paroi (110, 310).

12. Système pour ciment osseux selon la revendication 11, dans lequel le dispositif d'administration (500) comprend en outre
une gâchette (530, 730) supportée de manière pivotante par ledit boîtier (504, 704) et opérationnellement connectée audit mécanisme d'entraînement (506, 706) pour actionner ledit mécanisme d'entraînement (506, 706) lors de l'actionnement de ladite gâchette (530, 730) ; et
un système de liaison (508, 708) interconnectant opérationnellement ladite gâchette (530, 730) avec ledit mécanisme d'entraînement (506, 730) pour actionner ledit mécanisme d'entraînement (506, 706), ledit système de liaison (508, 730) comprenant une première liaison (532, 732) connectée de manière pivotante audit boîtier (504, 704) et une deuxième liaison (536, 736) interconnectant de manière pivotante ladite première liaison (532, 732) et ladite gâchette (530, 730) de telle sorte que l'actionnement de ladite gâchette (530, 730) déplace ladite deuxième liaison (536, 736) et ladite première liaison (532, 732) pour actionner ledit mécanisme d'entraînement (506, 706) et faire avancer ledit mécanisme d'entraînement (506, 706) pour décharger le ciment osseux hors de la cartouche (100).

13. Système pour ciment osseux selon la revendication 12, dans lequel ledit mécanisme d'entraînement (508) comprend en outre :
une tige d'entraînement (524) ; et
une première plaque de préhenseur (528) répondant à l'actionnement de ladite gâchette (530) dans lequel ladite première plaque de préhenseur (528) entoure ladite tige d'entraînement (524) et engage par frottement ladite tige d'entraînement (524) pour faire avancer ladite tige d'entraînement (524) lorsque ladite gâchette (530) est actionnée.

14. Système pour ciment osseux selon l'une des revendications 11 à 13, dans lequel lesdits éléments de libération (200) incluent chacun une surface de came (202) engageable par ledit mécanisme de libération (518) pour faire coulisser lesdits éléments de libération (200) radialement vers l'intérieur et déverrouiller ledit piston (114).

15. Système pour ciment osseux selon l'une des revendications 11 à 13, dans lequel ladite pluralité d'éléments de libération (400) sont en outre définis comme une pluralité de doigts (400) engageables par ledit mécanisme de libération (518) pour déplacer ladite pluralité de doigts (400) et déverrouiller ledit piston.
